# EUROPEAN PATENT APPLICATION

(11) **EP 4 151 236 A1**
(43) Date of publication of application: **22.03.2023**
(21) Application number: 21803785.1
(22) Date of filing: 13.05.2021
(51) Int. Cl.: A61K 48/00, A61K 47/60, A61K 47/58, A61K 47/54, A61K 47/69, A61K 31/713, A61K 9/19, A61P 3/04

(54) **COMPOSITION FOR PREVENTING OR TREATING OBESITY-RELATED DISEASE CONTAINING AMPHIREGULIN-SPECIFIC DOUBLE-STRANDED OLIGONUCLEOTIDE STRUCTURE**

(30) Priority: 14.05.2020 KR 20200057879
(71) Applicant: Bioneer Corporation, Daejeon 34302 (KR); Sirnagen Therapeutics Corporation, Daejeon 34302 (KR)
(72) Inventor: PARK, Jun Hong, Daejeon 34538 (KR); PARK, Han-Oh, Sejong-si 30151 (KR); YUN, Sung Il, Daejeon 34022 (KR); KIM, Tae Rim, Daejeon 35217 (KR); HWANG, Soo Hyun, Daejeon 34006 (KR); SONG, Kang, Daejeon 34401 (KR); JUNG, Sang Hyuk, Daejeon 34019 (KR); KIM, Jangseon, Daejeon 34011 (KR); LEE, Mi Sun, Daejeon 34011 (KR); CHOI, Soonja, Daejeon 34048 (KR); SON, Seung-Seob, Cheonan-si Chungcheongnam-do 31156 (KR)
(74) Representative: Mewburn Ellis LLP
(86) International application number: PCT/IB2021/054077
(87) International publication number: WO 2021/229479

(57) **Abstract**

The present invention relates to a double-stranded oligonucleotide capable of inhibiting amphiregulin expression in a very specific and highly efficient manner, preferably a double-stranded oligonucleotide comprising a sequence in the form of an RNA/RNA, DNA/DNA or DNA/RNA hybrid, and the use of a double-stranded oligonucleotide structure, which comprises the double-stranded oligonucleotide, and nanoparticles, for preventing or treating obesity.

## Description

### Technical Field

The present invention relates to a composition for preventing or treating obesity-related diseases comprising an amphiregulin-specific double-stranded oligonucleotide structure, and more particularly, to a double-stranded oligonucleotide capable of inhibiting amphiregulin expression in a very specific and highly efficiently manner, and a composition for preventing or treating obesity-related diseases comprising a double-stranded oligonucleotide structure, comprising the double-stranded oligonucleotide, and nanoparticles.

### Background Art

In 1995, Guo and Kemphues reported that not only sense RNA but also antisense RNA is effective in inhibiting gene expression in *C*. *elegans,* and since then, studies have been conducted to identify the cause thereof. In 1998, Fire et al. first described the phenomenon in which injection of double-stranded RNA (dsRNA) inhibits gene expression by specifically degrading the mRNA corresponding thereto. This phenomenon was named RNA interference (RNAi). RNAi, a process that is used to inhibit gene expression, may exhibit a distinct effect of inhibiting gene expression in a simple manner at low cost, and thus the application range of this technology has expanded.

Since this technology of inhibiting gene expression may regulate the expression of a specific gene, it may remove a specific gene related to cancer, genetic disease or the like at the mRNA level, and may be used as an important tool for the development of therapeutic agents for disease treatment and validation of targets. As conventional techniques for inhibiting target gene expression, techniques of introducing a transgene for a target gene have been disclosed. These techniques include a method of introducing a transgene in the antisense direction with respect to the promoter and a method of introducing a transgene in the antisense direction with respect to the promoter.

Such RNA therapy targeting RNA is a method of removing the function of the gene of interest using oligonucleotides against the target RNA, and may be considered different from conventional methods in which therapeutic agents such as antibodies and small molecules mainly target proteins. Approaches for targeting RNA are roughly classified into two types: double-stranded-RNA mediated RNAi, and an antisense oligonucleotide (ASO). Currently, clinical trials are being attempted by targeting RNA in various diseases.

An antisense oligonucleotide (hereinafter referred to as "ASO") is short synthetic DNA designed to bind to a target gene according to Watson-Crick base pairing, and may specifically inhibit the expression of a specific nucleotide sequence of a gene. Thus, the antisense oligonucleotide has been used to study the roles of genes and to develop therapeutic agents capable of treating diseases such as cancer at the molecular level. These ASOs have the advantage of being able to be easily produced by setting various targets for inhibiting gene expression, and studies have been conducted on the use of ASOs in order to inhibit oncogene expression and cancer cell growth. A process of inhibiting the expression of a specific gene by the ASO is accomplished either by binding the ASO to a complementary mRNA sequence to induce RNase H activity and remove the mRNA or by interfering with the formation and progression of a ribosome complex for protein translation. In addition, it has been reported that the ASO binds to genomic DNA to form a triple-helix structure, thus inhibiting gene transcription. The ASO has potential as described above, but in order to use the ASO in clinical practice, it is required that the stability of the ASO against nucleases be improved and that the ASO be efficiently delivered into a target tissue or cells so as to bind specifically to the nucleotide sequence of a target gene. In addition, the secondary and tertiary structures of genetic mRNA are important factors for specific binding of the ASO, and a region in which formation of the mRNA secondary structure decreases is very advantageous for the ASO to access. Thus, efforts have been made to effectively achieve gene-specific inhibition not only *in vitro* but also *in vivo* by systematically analyzing a region in which formation of the mRNA secondary structure decreases, prior to synthesizing the ASO. These ASOs are more stable than siRNA, a kind of RNA, and have the advantage of being readily soluble in water and physiological saline. To date, three ASOs have been approved by the Federal Drug Administration (FDA) (Jessica, C., J Postdoc Res, 4:35-50, 2016) .

Since the roles of RNA interference (hereinafter referred to as "RNAi") were found, it has been found that RNAi acts on sequence-specific mRNAs in various types of mammalian cells (Barik, S., J Mol. Med. (2005) 83: 764-773). When a long chain of double-stranded RNA is delivered into a cell, the delivered double-stranded RNA is converted into small interfering RNA (hereinafter referred to as "siRNA") processed to 21 to 23 base pairs (bp) by Dicer endonuclease. The siRNA binds to an RNA-induced silencing complex (RISC) and inhibits target gene expression in a sequence-specific manner through a process in which the guide (antisense) strand recognizes and degrades the target mRNA. Technology for inhibiting gene expression using SiRNA is used to inhibit target gene expression in target cells and to observe the resulting change, and is effectively used in studies to identify the function of a target gene in target cells. In particular, inhibiting the function of a target gene in infectious viruses or cancer cells may be effectively used to develop a treatment method for the disease of interest. As a result of conducting *in vitro* studies and *in vivo* studies using experimental animals, it has been reported that it is possible to inhibit target gene expression by siRNA.

Bertrand et al. reported that siRNA has a better inhibitory effect on mRNA expression *in vitro* and *in vivo* than an antisense oligonucleotide (ASO) against the same target gene, and that the effect is longer lasting. In addition, regarding the mechanism of action, siRNA regulates target gene expression in a sequence-specific manner by complementary binding to the target mRNA. Thus, siRNA has an advantage over conventional antibody-based drugs or chemical drugs (small-molecule drugs) in that the range of subjects to which the siRNA is applicable can be dramatically expanded.

siRNA has excellent effects and may be used in a wide range of applications, but in order for siRNA to be developed as a therapeutic agent, the *in vivo* stability of siRNA and the cell delivery efficiency thereof should be improved so that siRNA can be effectively delivered to the target cells. In order to improve *in vivo* stability and solve problems associated with non-specific innate immune stimulation of siRNA, studies thereon have been actively attempted by modifying some nucleotides of siRNA or the backbone thereof to have nuclease resistance, or using viral vectors, liposomes, or nanoparticles.

Delivery systems comprising a viral vector such as adenovirus or retrovirus have high transfection efficacy, but have high immunogenicity and oncogenicity. On the other hand, non-viral delivery systems containing nanoparticles have lower cell delivery efficiency than viral delivery systems, but have advantages, including high safety *in vivo,* target-specific delivery, efficient uptake and internalization of RNAi oligonucleotides into cells or tissues, and low cytotoxicity and immune stimulation. Thus, non-viral delivery systems are currently considered a more promising delivery method than viral delivery systems.

Among the non-viral delivery systems, methods that use nanocarriers are methods in which nanoparticles are formed using various polymers such as liposomes and cationic polymer complexes and in which siRNA is loaded into such nanoparticles (i.e., nanocarriers) and delivered to cells. Among the methods that use nanocarriers, frequently used methods include methods that use polymeric nanoparticles, polymer micelles, lipoplexes, and the like. Thereamong, lipoplexes are composed of cationic lipids, and function to interact with the anionic lipids of cellular endosomes to induce destabilization of the endosomes, thus allowing intracellular delivery of the exosomes.

In addition, it is known that the efficiency of siRNA in vivo can be increased by conjugating a chemical compound or the like to the end region of the passenger (sense) strand of the siRNA so as to impart improved pharmacokinetic characteristics thereto (J. Soutschek, Nature 11; 432(7014):173-8, 2004). In this case, the stability of the siRNA changes depending on the properties of the chemical compound conjugated to the end of the sense (passenger) or antisense (guide) strand of the siRNA. For example, siRNA conjugated with a polymer compound such as polyethylene glycol (PEG) interacts with the anionic phosphate group of siRNA in the presence of a cationic compound to form a complex, thereby providing a carrier having improved siRNA stability. In particular, micelles composed of a polymer complex have a very small size and a very uniform size distribution compared to other drug delivery systems such as microspheres or nanoparticles, and are spontaneously formed. Thus, these micelles have advantages in that the quality of the micelle formulation is easily managed and reproducibility thereof is easily secured.

In order to improve the intracellular delivery efficiency of siRNA, technology for ensuring the stability of the siRNA and increasing the cell membrane permeability of the siRNA using a siRNA conjugate, obtained by conjugating a hydrophilic compound (e.g., polyethylene glycol (PEG)), which is a biocompatible polymer, to the siRNA via a simple covalent bond or a linker-mediated covalent bond, has been developed (Korean Patent No. 883471). However, even when the siRNA is chemically modified and conjugated to polyethylene glycol (PEG) (PEGylation), it still has low stability *in vivo* and a disadvantage in that it is not easily delivered into a target organ. In order to overcome these disadvantages, a double-stranded oligo RNA structure has been developed, which comprises hydrophilic and hydrophobic compounds bound to an oligonucleotide, particularly double-stranded oligo RNA such as siRNA. This structure forms self-assembled nanoparticles, named SAMiRNA^{™} (Self Assembled Micelle Inhibitory RNA), by hydrophobic interaction of the hydrophobic compound (Korean Patent No. 1224828). The SAMiRNA^{™} technology has advantages over conventional delivery technologies in that homogenous nanoparticles having a very small size may be obtained.

Specifically, in the SAMiRNA^{™} technology, PEG (polyethylene glycol) or HEG (hexaethylene glycol) is used as the hydrophilic compound. PEG, a synthetic polymer, is generally used to increase the solubility of medical drugs, particularly proteins, and to regulate the pharmacokinetics of drugs. PEG is a polydisperse material, and a one-batch polymer is made up of different numbers of monomers, and thus exhibits a molecular weight distribution having a Gaussian curve. In addition, the homogeneity of a material is expressed as a polydispersity index (Mw/Mn). In other words, when PEG has a low molecular weight (3 to 5 kDa), it has a polydispersity index of about 1.01, and when PEG has a high molecular weight (20 kDa), it has a high a polydispersity index of about 1.2, indicating that the homogeneity of PEG decreases as the molecular weight thereof increases. Thus, when PEG is conjugated to a pharmaceutical drug, there is a disadvantage in that the polydisperse properties of PEG are reflected in the conjugate, and thus it is not easy to verify a single material. Due to this disadvantage, processes for the synthesis and purification of PEG have been improved in order to produce materials having a low polydispersity index. However, when PEG is conjugated to a compound having a low molecular weight, there are problems associated with the polydisperse properties of the compound, including a problem in that it is not easy to confirm whether conjugation was easily achieved.

Accordingly, in recent years, the SAMiRNA^{™} technology (that is, self-assembled nanoparticles) has been improved by forming the hydrophilic compound of the double-stranded RNA structure (constituting SAMiRNA^{™}) into basic unit blocks, each comprising 1 to 15 monomers having a uniform molecular weight, and if necessary, a linker, so that a suitable number of the blocks is used according to need. Thus, new types of delivery system technologies, which have small sizes and significantly improved polydisperse properties, compared to conventional SAMiRNA^{™}, have been developed. It is already known that, when siRNA is injected, the siRNA is rapidly degraded by various enzymes present in the blood, and thus the efficiency of delivery thereof to target cells or tissues is poor. As such, variation in stability and expression inhibition rate depending on target genes also appeared in improved SAMiRNA^{™}. Accordingly, in order to more stably and effectively inhibit the expression of a target gene using SAMiRNA^{™}, which is composed of improved self-assembled nanoparticles, the present inventors have attempted to enhance the expression inhibitory effect of SAMiRNA^{™} on the target gene and the stability of SAMiRNA^{™} by applying a double-stranded oligonucleotide comprising the DNA sequence of an ASO as the guide (sense) strand and an RNA sequence as the passenger (antisense sense) sequence.

Meanwhile, obesity is an important health problem worldwide, and may cause an increase in a number of complications such as heart disease, type 2 diabetes, and certain cancers.

One of the main causes of obesity is excessive accumulation of visceral fat in the body [Carr DB, Diabetes. 2004 Aug;53(8):2087-94 ; Bouchard C, Int J Obes Relat Metab Disord 1996;20:420-7]. Visceral fat refers to fat surrounding the internal organs, and visceral fat is mainly caused by a genetic factor [Rosenberg B, Panminerva Med 2005; 47:229-44], race, physical activity, lifestyle, and inflammatory factors [Deurenberg P, Int J Obes Relat Metab Disord 1998;22:1164-71]. In addition, it is known that the accumulation of visceral fat is severe in Asians among various races [WHO Expert Consultation. Lancet 2004;363:157-63; Hu FB, N Engl J Med 2001;345:790-7], and overeating or drinking, less physical activity [Wannamethee SG, Am J Clin Nutr 2003; 77:1312-7; Komiya H, Tohoku J Exp Med 2006;208:123-32], and smoking further increase visceral fat [Upadhyaya S, Adipocyte, 2014; 3(1): 39-45]. When visceral fat accumulates, the secretion of pro-inflammatory factors, such as interleukin-6, tumor necrosis factor-alpha, and monocyte chemoattractant protein-1, from visceral fat cells increases, causing various complications [Despres JP. Ann Med 2001;33:534-41]. Visceral fat causes metabolic abnormalities and cardiovascular disease [Matsuzawa Y, Obes Res 1995;3 Suppl 5:645-7]. Increased accumulation of visceral fat leads to increased insulin resistance, and if visceral fat outweighs subcutaneous fat, heart function decreases and hypertension and circulatory system disease occur [Schaffler, Nat Clin Pract Gastroenterol Hepatol 005;2:273-80]. Visceral fat also causes digestive disorders, and is known to cause fatty liver and nonalcoholic steatohepatitis [Busetto L, Diabetes Obes Metab 2005; 7:301-6]. Due to these factors, anti-inflammatory mechanisms are degraded as adiponectin is lowered, and fatty liver formation in the liver is promoted, causing non-alcoholic fatty liver. Visceral fat also causes many problems in respiratory diseases [Schapira DV, Cancer 1994; 74:632-9]. It can be seen that, as visceral fat outweighs subcutaneous fat, the expiratory reserve volume decreases, causing restrictive pulmonary ventilation dysfunction. Visceral fat is also known to increase the incidence of breast cancer. Visceral fat is also known to be associated with increased incidence of prostate cancer [Hsing AW, J Natl Cancer Inst 2001; 93:783-9] and colorectal cancer [Manson JE, N Engl J Med 1995; 333:677-85].

The treatment of visceral fat mainly consists of diet restriction, physical exercise and drug treatment, but the effect thereof is still insufficient [Diamantis T, Surg Obes Relat Dis, 2014; 10(1): 177-83 ; Kelley GA, J Obes, 2013; 2013783103 ; Rhines SD, S D Med, 2013; 66(11): 471, 73 ; Sharma M, Adolesc Health Med Ther, 2010; 19-19]. Therefore, reducing visceral fat can reduce the incidence of cardiovascular disease, metabolic disease, diabetes and other diseases, thereby preventing complications and improving quality of life.

Accordingly, the present inventors have conducted studies on treating obesity by reduction of visceral fat, and as a result, have found that the use of a structure comprising an amphiregulin-specific double-stranded oligonucleotide that specifically inhibits amphiregulin may significantly reduce visceral fat, including subcutaneous fat, in diabetic animal models.

In addition, the present inventors have found that the expression level of amphiregulin in epididymal fat is significantly high in a high-fat-diet-induced obese animal model, and when the expression level of amphiregulin in the obese animal model is reduced using the structure comprising an amphiregulin-specific double-stranded oligonucleotide according to the present invention, it is possible to significantly reduce body weight, subcutaneous fat weight, and visceral fat weight, suppress an increase in the size of adipose tissue cells, reduce expression of amphiregulin in adipose tissue, and inhibit fat accumulation in the liver, thereby completing the present invention related to the anti-obesity use of the structure comprising the amphiregulin-specific double-stranded oligonucleotide.

### Summary of the Invention

An object of the present invention is to provide a novel pharmaceutical composition for treating or preventing obesity.

To achieve the above object, the present invention provides a pharmaceutical composition for treating or preventing obesity comprising any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] A-X-R-Y-B

   wherein A represents a hydrophilic compound, B represents a hydrophobic compound, X and Y each independently represent a simple covalent bond or a linker-mediated covalent bond, and R represents the amphiregulin-specific double-stranded oligonucleotide (i); and
(iii) nanoparticles comprising the amphiregulin-specific double-stranded oligonucleotide structure (ii).

The present invention also provides a lyophilized formulation comprising the pharmaceutical composition.

The present invention also provides a method of preventing or treating obesity comprising a step of administering, to a subject in need of prevention or treatment of obesity, any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] A-X-R-Y-B

   wherein A represents a hydrophilic compound, B represents a hydrophobic compound, X and Y each independently represent a simple covalent bond or a linker-mediated covalent bond, and R represents the amphiregulin-specific double-stranded oligonucleotide (i); and
(iii) nanoparticles comprising the amphiregulin-specific double-stranded oligonucleotide structure (ii).

The present invention also provides a pharmaceutical composition for use in a method for preventing or treating obesity, the pharmaceutical composition comprising any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] A-X-R-Y-B

   wherein A represents a hydrophilic compound, B represents a hydrophobic compound, X and Y each independently represent a simple covalent bond or a linker-mediated covalent bond, and R represents the amphiregulin-specific double-stranded oligonucleotide (i); and
(iii) nanoparticles comprising the amphiregulin-specific double-stranded oligonucleotide structure (ii).

The present invention provides the use of any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] A-X-R-Y-B

   wherein A represents a hydrophilic compound, B represents a hydrophobic compound, X and Y each independently represent a simple covalent bond or a linker-mediated covalent bond, and R represents the amphiregulin-specific double-stranded oligonucleotide (i); and
(iii) nanoparticles comprising the amphiregulin-specific double-stranded oligonucleotide structure (ii), in manufacture of a medicament for preventing or treating obesity.

### Brief Description of Drawings

FIG. 1 shows the results of screening 1,257 SAMiRNAs targeting human amphiregulin.
FIG. 2 shows the nanoparticle size distribution of double-stranded oligo DNA/RNA hybrids comprising a selected amphiregulin-specific double-stranded oligonucleotide. (a): SAMi- AREG#10; (b): SAMi-AREG#11; and (c): SAMi-AREG#12.
FIG. 3 depicts graphs showing the results of quantitatively analyzing amphiregulin mRNA expression levels in Example 4. Here, the lung cancer cell line A549 was treated with different concentrations (200 and 600 nM) of a SAMiRNA having each of the sequences of SEQ ID NOs: 1 to 14 of the present invention as a sense strand, and relative amphiregulin mRNA expression levels (%) in the cells were analyzed.
FIG. 4 depicts graphs showing the results of quantitatively analyzing amphiregulin mRNA expression levels in Example 5. Here, the lung cancer cell line A549 was treated with different concentrations (12.5 nM, 25 nM, 50 nM, 100 nM, 200 nM, 600 nM and 1,200 nM) of a SAMiRNA having the sequence of SEQ ID NO: 10 of the present invention as a sense strand, relative amphiregulin mRNA expression levels (%) in the cells were analyzed (FIG. 4a), and the IC₅₀ value of the SAMiRNA was determined (FIG. 4b).
FIG. 5 depicts graphs showing the results of quantitatively analyzing amphiregulin mRNA expression levels in Example 5. Here, the lung cancer cell line A549 was treated with different concentrations (12.5 nM, 25 nM, 50 nM, 100 nM, 200 nM, 600 nM and 1,200 nM) of a SAMiRNA having the sequence of SEQ ID NO: 11 of the present invention as a sense strand, relative amphiregulin mRNA expression levels (%) in the cells were analyzed (FIG. 5a), and the IC₅₀ value of the SAMiRNA was determined (FIG. 5b).
FIG. 6 depicts graphs showing the results of quantitatively analyzing amphiregulin mRNA expression levels in Example 5. Here, the lung cancer cell line A549 was treated with different concentrations (12.5 nM, 25 nM, 50 nM, 100 nM, 200 nM, 600 nM and 1,200 nM) of a SAMiRNA having the sequence of SEQ ID NO: 12 of the present invention as a sense strand, relative amphiregulin mRNA expression levels (%) in the cells were analyzed (FIG. 6a), and the IC₅₀ value of the SAMiRNA was determined (FIG. 6b).

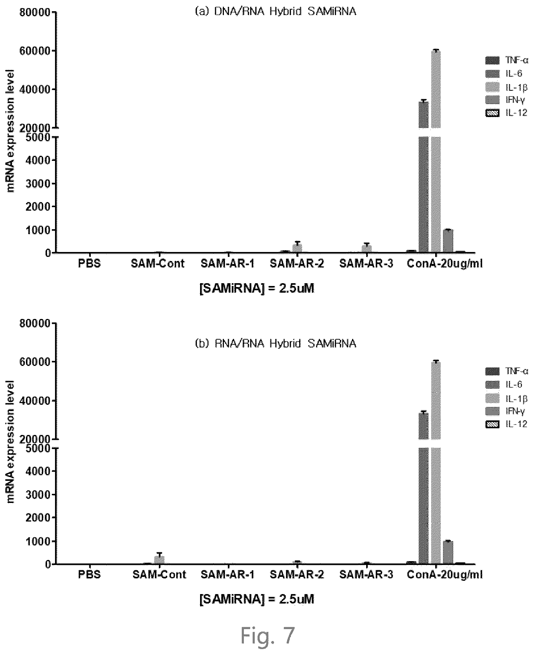

FIG. 7 depicts graphs showing the results of an innate immune response test for amphiregulin candidate sequences in Example 6. Here, peripheral blood mononuclear cells (PBMCs) were treated with 2.5 µM of amphiregulin-specific SAMiRNA having each of the sequences of SEQ ID NOs: 10 (AR-1), 11 (AR-2) and 12 (AR3) of the present invention as a sense strand, the relative increases in mRNA expression levels of innate immune-related cytokines by amphiregulin-specific SAMiRNA were analyzed, and *in vitro* cytotoxicity was evaluated using the human peripheral blood mononuclear cells. (a): DNA/RNA hybrid SAMiRNA, and (b) : RNA/RNA hybrid SAMiRNA.

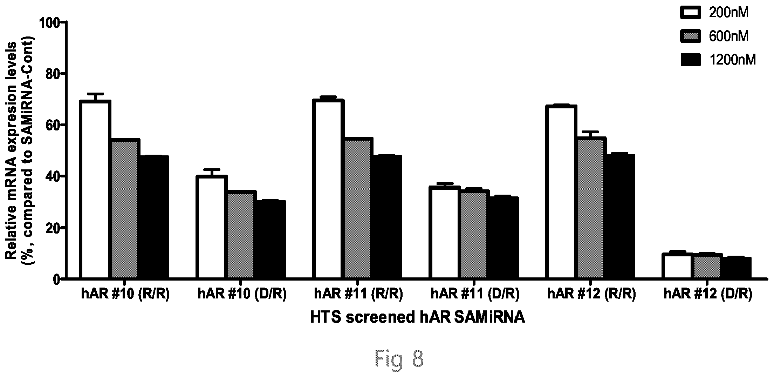

FIG. 8 is a graph showing the results of quantitatively analyzing amphiregulin mRNA expression levels in Example 7. Here, the relative mRNA expression levels (%) of amphiregulin by a double-stranded oligo DNA/RNA hybrid and an RNA/RNA hybrid, each comprising a selected amphiregulin-specific SAMiRNA, were comparatively analyzed. Specifically, the lung cancer cell line A549 treated with different concentrations (200 nM, 600 nM and 1,200 nM) of SAMiRNA having each of the sequences of SEQ ID NOs: 10 (AR-1), 11 (AR-2) and 12 (AR-3) of the present invention as a sense strand, and relative amphiregulin mRNA expression levels (%) were comparatively analyzed.
FIG. 9 shows the results of screening 237 SAMiRNAs, which target mouse amphiregulin, and 9 candidate sequences selected therefrom.
FIG. 10A depicts graphs showing the results of quantitatively analyzing amphiregulin mRNA expression levels in Example 8. Here, the mouse lung fibroblast cell line MLg was treated with different concentrations (200 and 500 nM) of SAMiRNA having each of the sequences of SEQ ID NOs: 19, 20 and 21 of the present invention as a sense strand, and relative amphiregulin mRNA expression levels (%) in the cells were analyzed.
FIG. 10B depicts graphs showing the results of quantitatively analyzing amphiregulin mRNA expression levels in Example 7. Here, the mouse lung epithelial cell line LA-4 was treated with different concentrations (200 and 500 nM) of SAMiRNA having each of the sequences of SEQ ID NOs: 19, 20 and 21 of the present invention as a sense strand, and relative amphiregulin mRNA expression levels (%) in the cells were analyzed.
FIG. 11 is a graph showing changes in body weight of a control group and experimental groups in a mouse animal model experiment according to the present invention.
FIG. 12 is a graph showing changes in food uptake of a control group and experimental groups in a mouse animal model experiment according to the present invention.
FIG. 13 is a graph showing changes in water intake of a control group and experimental groups in a mouse animal model experiment according to the present invention.
FIG. 14 shows the results of measuring the subcutaneous fat ratio of each of a control group and experimental groups in a mouse animal model experiment according to the present invention.
FIG. 15 shows the results of measuring the visceral fat ratio of each of a control group and experimental groups in a mouse animal model experiment according to the present invention.
FIG. 16 depicts photographs showing the visceral fat-reducing effect of the structure according to the present invention in a mouse animal model experiment according to the present invention.
FIG. 17 shows the results of measuring the fasting blood glucose level in whole blood 8 weeks before sacrifice of a control group and experimental groups in the mouse animal model experiment according to the present invention.
FIG. 18 shows the results of measuring serum glucose levels 8 weeks before sacrifice of a control group and experimental groups in the mouse animal model experiment according to the present invention.
FIG. 19 is a graph showing the results of analyzing the expression level of amphiregulin in epididymal fat after sacrificing a control group and an experimental group after 12 weeks of high-fat feeding in a high-fat-diet obese mouse model experiment according to the present invention.
FIG. 20 is a graph showing changes in body weight of a control group and an experimental group in a high-fat diet obese mouse model experiment according to the present invention.
FIG. 21a is a graph showing the average food intake of each of a control group and an experimental group in a high-fat diet obese mouse model experiment according to the present invention.
FIG. 21b is a graph showing the average water intake of each of a control group and an experimental group in a high-fat diet obese mouse model experiment according to the present invention.
FIG. 22 is a graph showing the food efficiency ratio (%) of each of a control group and an experimental group in a high-fat diet obese mouse model experiment according to the present invention.
FIG. 23a shows the results of measuring the subcutaneous fat pad (SFP), epididymal fat pad (EFP), perirenal fat pad (PFP), mesenteric fat pad (MFP) subcutaneous fat pad (SFP), epididymal fat pad (EFP), perirenal fat pad (PFP), and mesenteric fat pad (MFP) weights of each of a control group and an experimental group in a high-fat diet obese mouse model experiment according to the present invention.

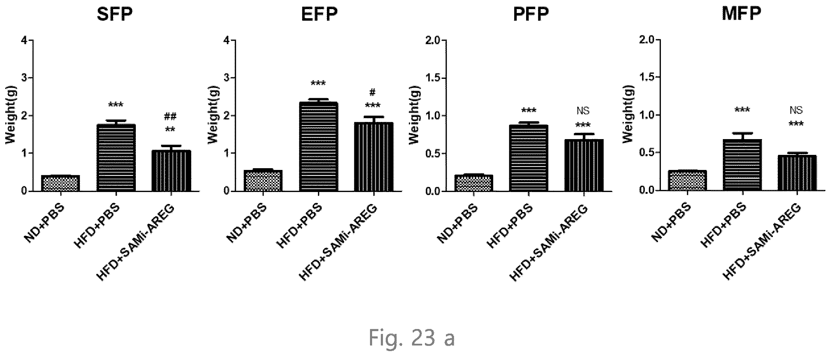

FIG. 23a shows the ratios (%) obtained by dividing the subcutaneous fat pad (SFP), epididymal fat pad (EFP), perirenal fat pad (PFP), mesenteric fat pad (MFP) subcutaneous fat pad (SFP), epididymal fat pad (EFP), perirenal fat pad (PFP), and mesenteric fat pad (MFP) weights of each of a control group and an experimental group by the body weight in a high-fat diet obese mouse model experiment according to the present invention.

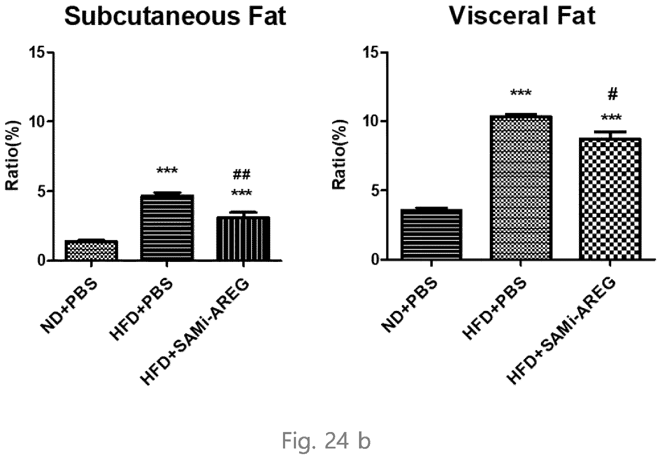

FIG. 24b shows the results of measuring the ratio of each of subcutaneous fat and visceral fat to total mouse weight in each of a control group and an experimental group in a high-fat diet obese mouse model experiment according to the present invention.

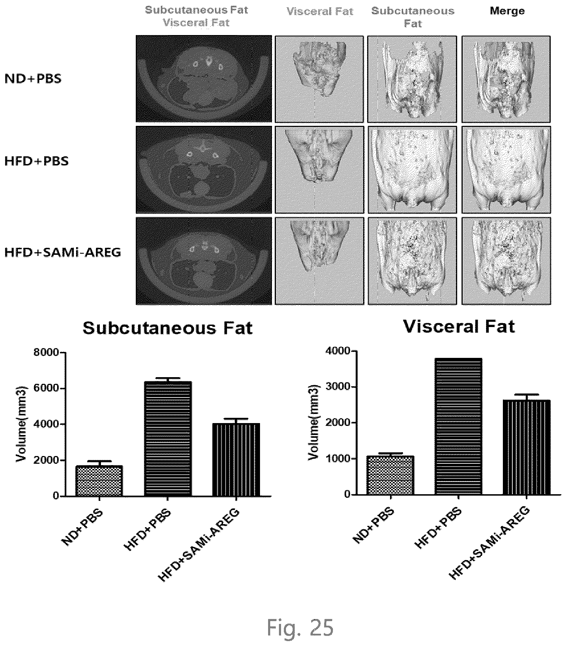

FIG. 25 depicts micro-CT images comparing the effect of reducing subcutaneous fat and visceral fat between a control group and an experimental group in a high-fat diet obese mouse model experiment according to the present invention, and depicts graphs showing the volume of each of subcutaneous fat and visceral fat.

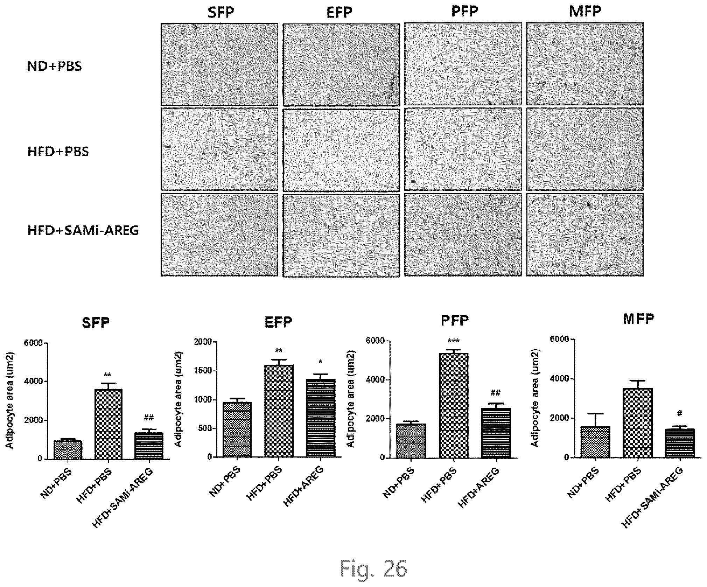

FIG. 26 depicts histological images comparing the effect of reducing subcutaneous fat pad (SFP), epididymal fat pad (EFP), perirenal fat pad (PFP) and mesenteric fat pad (MFP) between a control group and an experimental group in a high-fat diet obese mouse model experiment according to the present invention, and depicts graphs showing the area of adipocytes in each group.

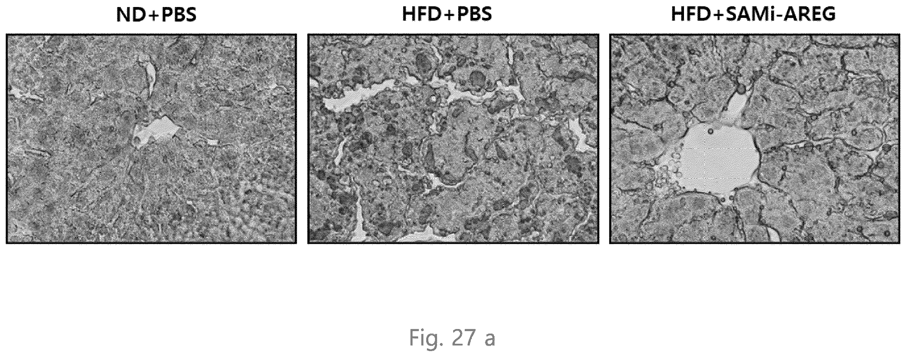

FIG. 27a depicts images comparing the effect of reducing fat accumulation in liver tissue between a control group and an experimental group in a high-fat diet obese mouse model experiment according to the present invention.

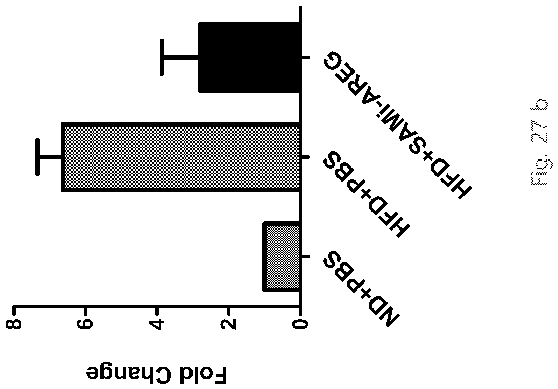

FIG. 27b is a quantitative graph comprising the effect of reducing fat accumulation in liver tissue between a control group and an experimental group weight in a high-fat diet obese mouse model experiment according to the present invention.

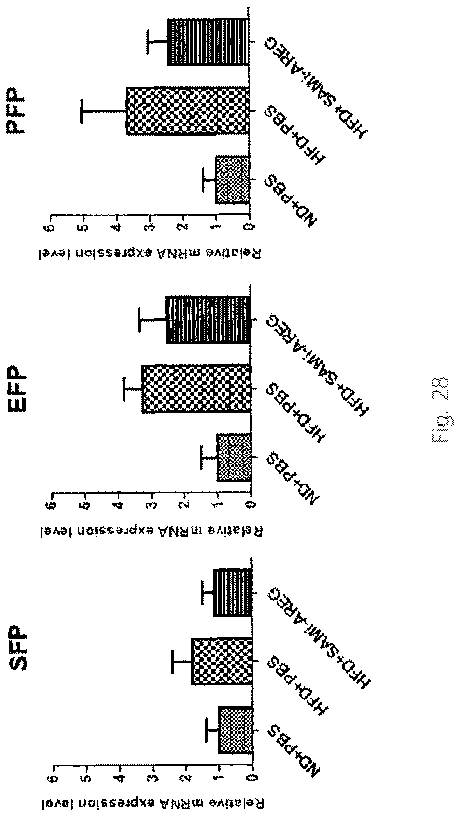

FIG. 28 depicts graphs comparing the effect of reducing amphiregulin mRNA levels in subcutaneous fat pad (SFP), epididymal fat pad (EFP) and perirenal fat pad (PFP) between a control group and an experimental group weight in a high-fat diet obese mouse model experiment according to the present invention.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, it has been found that, when an amphiregulin-specific double-stranded oligonucleotide structure is administered to an animal model of type 2 diabetes, it exhibits the effect of significantly reducing subcutaneous fat and visceral fat, indicating that the amphiregulin-specific double-stranded oligonucleotide structure may be used as a composition for preventing or treating obesity.

In addition, in the present invention, it has been found that, when the amphiregulin-specific double-stranded oligonucleotide structure is administered to an obesity-induced animal model, it exhibits the effects of losing weight, reducing food efficiency ratio, reducing subcutaneous fat, reducing visceral fat, reducing adipocyte area, and inhibiting liver adipogenesis.

Therefore, in one aspect, the present invention is directed to a pharmaceutical composition for treating or preventing obesity comprising any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] A-X-R-Y-B

   wherein A represents a hydrophilic compound, B represents a hydrophobic compound, X and Y each independently represent a simple covalent bond or a linker-mediated covalent bond, and R represents the amphiregulin-specific double-stranded oligonucleotide (i); and
(iii) nanoparticles comprising the amphiregulin-specific double-stranded oligonucleotide structure (ii).

The sequences of SEQ ID NOs: 10, 11 and 12, which are each comprised in a preferred double-stranded oligonucleotide provided according to the present invention, are as follows:
5'-CACCTACTCTGGGAAGCGT-3' (SEQ ID NO: 10)
5'-ACCTACTCTGGGAAGCGTG-3' (SEQ ID NO: 11)
5'-CTGGGAAGCGTGAACCATT-3' (SEQ ID NO: 12)

As used herein, the term "double-stranded oligonucleotide" is intended to include all materials having general RNAi (RNA interference) activity, and it will be obvious to those skilled in the art that an mRNA-specific double-stranded oligonucleotide that encodes the amphiregulin protein also includes amphiregulin-specific shRNA or the like. That is, the oligonucleotide may be siRNA, shRNA or miRNA.

In addition, it will be obvious to those skilled in the art that amphiregulin-specific siRNA which comprises a sense strand and an antisense strand, or an antisense oligonucleotide, each comprising a sequence resulting from substitution, deletion or insertion of one or more nucleotides in a sense strand comprising any one sequence selected from the group consisting of SEQ ID NOs: 10, 11 and 12, or an antisense strand complementary thereto, is also within the scope of the present invention, as long as the specificity thereof to amphiregulin is maintained.

In the present invention, the sense or antisense strand may be independently DNA or RNA. In addition, the sense and antisense strands may be in the form of a hybrid in which the sense strand is DNA and the antisense strand is RNA or the sense strand is RNA and the antisense strand is DNA.

In the present invention, SEQ ID NOs: 10, 11 and 12 are set forth in the form of DNA, but when the form of RNA is used, the sequences of SEQ ID NOs: 10, 11 and 12 may be RNA sequences corresponding thereto, that is, sequences in which T is substituted with U.

In addition, the double-stranded oligonucleotide according to the present invention includes not only the case where the sense strand of the sequence is fully complementary to (perfect matches) the binding site of the amphiregulin gene, but also the case where the sense strand is partially complementary (mismatch) to the binding site, as long as the specificity to amphiregulin is maintained.

The double-stranded oligonucleotide according to the present invention may comprise, at the 3' end of one or both strands, an overhang comprising one or more unpaired nucleotides.

In the present invention, the sense strand or the antisense strand preferably consists of 19 to 31 nucleotides, without being limited thereto.

In the present invention, the double-stranded oligonucleotide comprising a sense strand, which comprises any one sequence selected from the group consisting of SEQ ID NOs: 10, 11 and 12, and an antisense strand comprising a sequence complementary thereto, may be specific to amphiregulin, without being limited thereto.

In the present invention, the sense strand or antisense strand of the double-stranded oligonucleotide may comprise various chemical modifications in order to increase the *in vivo* stability thereof or impart nuclease resistance and reduce non-specific immune responses. The chemical modification may be one or more selected from, without limitation to, the group consisting of the following chemical modifications: modification in which an OH group at the 2' carbon position of a sugar structure in one or more nucleotides is substituted with any one selected from the group consisting of a methyl group (-CH₃), a methoxy group (-OCH₃), an amine group (-NH₂), fluorine (-F), a -O-2-methoxyethyl group, an -O-propyl group, an -O-2-methylthioethyl group, an -0-3-aminopropyl group, an -O-3-dimethylaminopropyl group, an -O-N-methylacetamido group, and an -O-dimethylamidooxyethyl group; modification in which oxygen in a sugar structure in nucleotides is substituted with sulfur; modification of a bond between nucleotides into any one bond selected from the group consisting of a phosphorothioate bond, a boranophophate bond and a methyl phosphonate bond; modification to PNA (peptide nucleic acid), LNA (locked nucleic acid) or UNA (unlocked nucleic acid); and modification to a DNA-RNA hybrid (Ann. Rev. Med. 55, 61-65 2004; US 5,660,985; US 5,958,691; US 6,531,584; US 5,808,023; US 6,326,358; US 6,175,001; Bioorg. Med. Chem. Lett. 14:1139-1143, 2003; RNA, 9:1034-1048, 2003; Nucleic Acid Res. 31:589-595, 2003; Nucleic Acids Research, 38(17) 5761-773, 2010; Nucleic Acids Research, 39(5):1823-1832, 2011).

In the present invention, one or more phosphate groups, preferably one to three phosphate groups, may be bound to the 5' end of the antisense strand of the double-stranded oligonucleotide.

In another aspect, the present invention is directed to a double-stranded oligonucleotide structure comprising a structure represented by the following Structural Formula (1), wherein A represents a hydrophilic compound, B represents a hydrophobic compound, X and Y each independently represent a simple covalent bond or a linker-mediated covalent bond, and R represents a double-stranded oligonucleotide.

In a preferred embodiment, the double-stranded oligonucleotide structure comprising an amphiregulin-specific sequence according to the present invention preferably has a structure represented by the following Structural Formula (1):

[Structural Formula (1)] A-X-R-Y-B

wherein A represents a hydrophilic compound, B represents a hydrophobic compound, X and Y each independently represent a simple covalent bond or a linker-mediated covalent bond, and R represents an amphiregulin-specific double-stranded oligonucleotide.

The double-stranded oligonucleotide according to the present invention is preferably in the form of a DNA-RNA hybrid, siRNA (short interfering RNA), shRNA (short hairpin RNA) or miRNA (microRNA), without being limited thereto, and may also include a single-stranded miRNA inhibitor that may act as an antagonist against miRNA.

Hereinafter, the double-stranded oligonucleotide according to the present invention will be described with a focus on RNA, but it is will be obvious to those skilled in the art that the present invention may also be applied to other double-stranded oligonucleotides having the same characteristics as the double-stranded oligonucleotide of the present invention.

More preferably, the double-stranded oligonucleotide structure comprising the amphiregulin-specific double-stranded oligonucleotide according to the present invention has a structure represented by the following Structural Formula (2):

[Structural Formula (2)] A-X-S-Y-B AS

wherein A, B, X and Y are as defined in Structural Formula (1) above, S represents the sense strand of the amphiregulin-specific double-stranded oligonucleotide, and AS represents the antisense strand of the amphiregulin-specific double-stranded oligonucleotide.

More preferably, the double-stranded oligonucleotide structure comprising the amphiregulin-specific double-stranded oligonucleotide has a structure represented by the following Structural Formula (3) or (4) :

[Structural Formula (3)] A - X - 5' S 3' - Y - B AS

[Structural Formula (4)] A - X - 3' S S' - Y - B AS

wherein A, B, S, AS, X and Y are as defined in Structural Formula (2) above, and 5' and 3' represent the 5' end and 3' end, respectively, of the sense strand of the amphiregulin-specific double-stranded oligonucleotide.

The hydrophilic compound may be selected from the group consisting of polyethylene glycol (PEG), polyvinylpyrrolidone, and polyoxazoline, without being limited thereto.

It will be obvious to those skilled in the art to which the present invention pertains that one to three phosphate groups may be bound to the 5' end of the antisense strand of the double-stranded oligonucleotide RNA structure comprising the amphiregulin-specific siRNA shown in Structural Formula (1) to Structural Formula (4) and that shRNA may be used in place of the RNA.

The hydrophilic compound in Structural Formula (1) to Structural Formula (4) above is preferably a polymer compound having a molecular weight of 200 to 10,000, more preferably a polymer compound having a molecular weight of 1,000 to 2,000. For example, as the hydrophilic polymer compound, it is preferable to use a nonionic hydrophilic polymer compound such as polyethylene glycol, polyvinyl pyrrolidone or polyoxazoline, without being necessarily limited.

In particular, the hydrophilic compound (A) in Structural Formula (1) to Structural Formula (4) may be used in the form of hydrophilic blocks as shown in the following Structural Formula (5) or (6), and a suitable number (n in Structural Formula (5) or (6)) of such hydrophilic blocks may be used as required, thereby overcoming the problems associated with polydisperse properties that may occur when general synthetic polymer compounds are used:

[Structural Formula (5)] (A'ₘ-J)ₙ

[Structural Formula (6)] (J-A' ₘ)ₙ

wherein A' represents a hydrophilic monomer, J represents a linker that connects m hydrophilic monomers together or connects m hydrophilic monomers with the double-stranded oligonucleotide, m is an integer ranging from 1 to 15, n is an integer ranging from 1 to 10, and a repeat unit represented by (A'ₘ-J) or (J-Aₘ') corresponds to the basic unit of the hydrophilic block.

When the hydrophilic block shown in Structural Formula (5) or (6) above is used, the double-stranded oligonucleotide structure comprising the amphiregulin-specific oligonucleotide according to the present invention may have a structure represented by the following Structural Formula (7) or (8):

[Structural Formula (7)] (A'ₘ-J)ₙ-X-R-Y-B

[Structural Formula (8)] (J-A'ₘ) ₙ-X-R-Y-B

wherein X, R, Y and B are as defined in Structural Formula (1) above, and A', J, m and n are as defined in Structural Formulas (5) and (6) above.

As the hydrophilic monomer (A') in Structural Formulas (5) and (6) above, one selected from among nonionic hydrophilic polymers may be used without limitation, as long as it is compatible with the purpose of the present invention. Preferably, a monomer selected from among compound (1) to compound (3) set forth in Table 1 below may be used. More preferably, a monomer of compound (1) may be used. In compound (1), G may preferably be selected from among O, S and NH.

In particular, among hydrophilic monomers, the monomer represented by compound (1) is very suitable for the production of the structure according to the present invention, because the monomer has advantages in that various functional groups may be introduced to the monomer, and the monomer induces little immune response by having good *in vivo* affinity and excellent biocompatibility, may increase the *in vivo* stability of the double-stranded oligonucleotide comprised in the structure represented by Structural Formula (7) or (8), and may increase the delivery efficiency of the double-stranded oligonucleotide.

**[Table 1] Structure of hydrophilic monomers used in the present invention**

| Compound (1) | Compound (2) | Compound (3) |
|---|---|---|
| 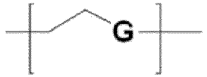 | 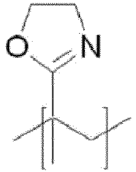 | 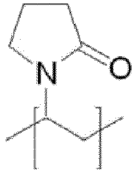 |
| G is O, S or NH | | |

The total molecular weight of the hydrophilic compound in Structural Formula (5) to Structural Formula (8) is preferably in the range of 1,000 to 2,000. Thus, for example, when compound (1) in Structural Formula (7) and Structural Formula (8) is hexaethylene glycol, that is, a compound in which G is O and m is 6, the repeat number (n) is preferably 3 to 5, because the hexaethylene glycol space has a molecular weight of 344. Particularly, the present invention is characterized in that a suitable number (represented by n) of repeat units of the hydrophilic group (hydrophilic blocks) represented by (A'ₘ-J) or (J-A'ₘ)ₙ in Structural Formula (5) and Structural Formula (6) may be used as required. The hydrophilic monomer J and linker J comprised in each hydrophilic block may be the same or different between the hydrophilic blocks. In other words, when 3 hydrophilic blocks are used (n = 3), the hydrophilic monomer of compound (1), the hydrophilic monomer of compound (2) and the hydrophilic monomer of compound (3) may be used in the first, second and third blocks, respectively, suggesting that different monomers may be used in all hydrophilic blocks. Alternatively, any one hydrophilic monomer selected from among the hydrophilic monomers of compounds (1) to (3) may also be used in all of the hydrophilic blocks. Similarly, as the linker that mediates the bonding of the hydrophilic monomer, the same linker may be used in the hydrophilic blocks, or different linkers may also be used in the hydrophilic blocks. In addition, m, which is the number of hydrophilic monomers, may also be the same or different between the hydrophilic blocks. In other words, in the first hydrophilic block, three hydrophilic monomers are connected (m=3), and in the second hydrophilic block, five hydrophilic monomers are connected (m=5), and in the third hydrophilic block, four hydrophilic monomers are connected (m=4), suggesting that different numbers of hydrophilic monomers may be used in the hydrophilic blocks. Alternatively, the same number of hydrophilic monomers may also be used in all hydrophilic blocks.

In addition, in the present invention, the linker (J) is preferably selected from the group consisting of - PO₃⁻-, - SO₃-, and -CO₂-, without being limited thereto. It will be obvious to those skilled in the art that any linker selected in consideration of the hydrophilic monomer that is used may be used, as long as it is compatible with the purpose of the present invention.

The hydrophobic compound (B) in Structural Formula (1) to Structural Formula (4), Structural Formula (7) and Structural Formula (8) functions to form nanoparticles composed of the oligonucleotide structure shown in Structural Formula (1) to Structural Formula (4), Structural Formula (7) and Structural formula (8), through hydrophobic interactions. The hydrophobic compound preferably has a molecular weight of 250 to 1,000, and may be any one selected from the group consisting of a steroid derivative, a glyceride derivative, glycerol ether, polypropylene glycol, a C₁₂-C₅₀ unsaturated or saturated hydrocarbon, diacyl phosphatidylcholine, a fatty acid, a phospholipid, lipopolyamine, a lipid, tocopherol, and tocotrienol, without being limited thereto. It will be obvious to those skilled in the art that any hydrophobic compound may be used, as long as it is compatible with the purpose of the present invention.

The steroid derivative may be selected from the group consisting of cholesterol, cholestanol, cholic acid, cholesteryl formate, cholestanyl formate, and cholesteryl amine, and the glyceride derivative may be selected from among mono-, di-, and tri-glycerides and the like. Here, the fatty acid of the glyceride is preferably a C₁₂-C₅₀ unsaturated or saturated fatty acid.

In particular, among the hydrophobic compounds, a saturated or unsaturated hydrocarbon or cholesterol is preferably used because it may be easily bound in a step of synthesizing the double-stranded oligonucleotide structure according to the present invention. Most preferably, a C₂₄ hydrocarbon, particularly a hydrophobic hydrocarbon containing a disulfide bond, is used.

The hydrophobic compound may be bound to the distal end of the hydrophilic compound, and may be bound to any position on the sense or antisense strand of the double-stranded oligonucleotide

The hydrophilic compound or hydrophobic compound in Structural Formulas (1) to (4), (7) and (8) according to the present invention is bound to the amphiregulin-specific oligonucleotide by a single covalent bond or a linker-mediated covalent bond (X or Y). The linker that mediates the covalent bond is covalently bound to the hydrophilic or hydrophobic compound at the end of the amphiregulin-specific oligonucleotide, and is not specifically limited, as long as it provides a degradable bond in a specific environment if required. Therefore, the linker that is used in the present invention may be any compound that is bound in order to activate the amphiregulin-specific double-stranded oligonucleotide and/or the hydrophilic (or hydrophobic) compound in the process of producing the double-stranded oligonucleotide structure according to the present invention. The covalent bond may be either one of a non-degradable bond and a degradable bond. Here, examples of the non-degradable bond include, but are not limited to, an amide bond and a phosphate bond, and examples of the degradable bond include, but are not limited to, a disulfide bond, an acid-degradable bond, an ester bond, an anhydride bond, a biodegradable bond, and an enzyme-degradable bond.

In addition, as the amphiregulin-specific double-stranded oligonucleotide represented by R (or S and AS) in Structural Formulas (1) to (4), (7) and (8), any double-stranded oligonucleotide having the property of binding specifically to the mRNA of amphiregulin may be used without limitation. Preferably, the amphiregulin-specific double-stranded oligonucleotide according to the present invention comprises a sense strand comprising any one sequence selected from among SEQ ID NOs: 10, 11 and 12, and an antisense strand comprising a sequence complementary to that of the sense strand.

In addition, in the double-stranded oligonucleotide structure comprising the amphiregulin-specific double-stranded oligonucleotide according to the present invention, an amine or polyhistidine group may additionally be introduced to the distal end of the hydrophilic compound bound to the oligonucleotide in the structure.

This facilitates intracellular uptake and endosomal escape of a carrier comprising the double-stranded oligonucleotide structure comprising the amphiregulin-specific double-stranded oligonucleotide according to the present invention, and it has already been reported that the introduction of an amine group and a polyhistidine group may be used to facilitate the intracellular uptake and endosomal escape of carriers such as quantum dots, dendrimers or liposomes.

Specifically, it is known that a primary amine group introduced to the end or outside of a carrier is protonated at biological pH while forming a conjugate by interaction with a negatively charged gene, and that endosomal escape is facilitated due to an internal tertiary amine having a buffering effect at low pH after intracellular uptake, whereby the carrier can be protected from lysosomal degradation (Gene Delivery and Expression Inhibition Using Polymer-Based Hybrid Material, Polymer Sci. Technol., Vol. 23, No. 3, pp 254-259) .

In addition, it is known that histidine, a non-essential amino acid, has an imidazole ring (pKa = 6.04) at the residue (-R) thereof, and thus has an effect of increasing buffering capacity in endosomes and lysosomes, and thus histidine modification may be used in non-viral gene carriers, including liposomes, in order to increase endosomal escape efficiency (Novel histidine-conjugated galactosylated cationic liposomes for efficient hepatocyte selective gene transfer in human hepatoma HepG2 cells. J. Controlled Release 118, pp. 262-270).

The amine group or polyhistidine group may be connected to the hydrophilic compound or the hydrophilic block by one or more linkers.

When the amine group or polyhistidine group is introduced to the hydrophilic compound of the double-stranded oligonucleotide structure represented by Structural Formula (1) according to the present invention, the RNA structure may have a structure shown in the following Structural Formula (9):

[Structural Formula (9)] P-J₁-J₂-A-X-R-Y-B

wherein A, B, R, X and Y are as defined in Structural Formula (1) above, P represents an amine group or a polyhistidine group, and J₁ and J₂ are linkers each of which may be independently selected from among a simple covalent bond, PO₃⁻, SO₃, CO₂, a C₂₋₁₂ alkyl, alkenyl and alkynyl, without being limited thereto. It will be obvious to those skilled in the art that any linkers selected in consideration of the hydrophilic compound used herein may be used as J₁ and J₂, as long as they are compatible with the purpose of the present invention.

Preferably, when an amine group is introduced, J₂ is a simple covalent bond or PO₃⁻, and J₁ is a C₆ alkyl, without being limited thereto

In addition, when a polyhistidine group is introduced, it is preferred that J₂ in Structural Formula (9) be a simple covalent bond or PO₃⁻, and that J₁ be compound (4), without being limited thereto.

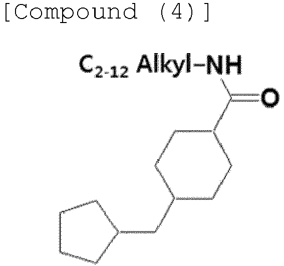

In addition, when the hydrophilic compound of the double-stranded oligonucleotide structure shown in Structural Formula (9) is the hydrophilic block represented by Structural Formula (5) or (6) and an amine group or a polyhistidine group is introduced thereto, the double-stranded oligonucleotide structure may have a structure represented by the following Structural Formula (10) or (11):

[Structural Formula (10)] P-J₁-J₂- (A'ₘ-J)ₙ -X-R-Y-B

[Structural Formula (11)] P-J₁-J₂- (J-A'ₘ)ₙ -X-R-Y-B

wherein X, R, Y, B, A', J, m and n are as defined in Structural Formula (5) or (6) above, and P, J₁ and J₂ are as defined in Structural Formula (9) above.

In particular, the hydrophilic compound in Structural Formula (10) and Structural Formula (11) is preferably bound to the 3' end of the sense strand of the amphiregulin-specific double-stranded oligonucleotide. In this case, Structural Formula (9) to Structural Formula (11) may correspond to the following Structural Formula (12) to Structural Formula (14):

[Structural Formula (12)] P-J₁-J₂-A-X-3' S 5'-Y-B AS

[Structural Formula (13)] P-J₁-J₂(A'ₘ-J)ₙ-X-3' S 5'-Y-B AS

[Structural Formula (14)] P-J₁-J₂-(J-A'ₘ)ₙ-X-3' S 5'-Y-B AS

wherein X, R, Y, B, A, A' J, m, n, P, J₁ and J₂ are as defined in Structural Formula (9) to Structural Formula (11) above, and 5' and 3' represent the 5' end and the 3' end of the sense strand of the amphiregulin-specific double-stranded oligonucleotide.

An amine group that may be introduced in the present invention may be a primary, secondary or tertiary amine group. In particular, a primary amine group is preferably used. The introduced amine group may be present as an amine salt. For example, a salt of the primary amine group may be present as NH₃⁺.

In addition, a polyhistidine group that may be introduced in the present invention preferably comprises 3 to 10 histidines, more preferably 5 to 8 histidines, and most preferably 6 histidines. In addition to histidine, one or more cysteines may be included.

Meanwhile, when a targeting moiety is provided in the double-stranded oligonucleotide structure comprising the amphiregulin-specific oligonucleotide according to the present invention and nanoparticles formed therefrom, it may promote the efficient delivery of the structure or nanoparticles to target cells, so that the structure or nanoparticles may be delivered to the target cells even at a relatively low concentration, thus exhibiting a strong effect of regulating target gene expression. In addition, the targeting moiety may prevent non-specific delivery of the amphiregulin-specific double-stranded oligonucleotide to other organs and cells.

Accordingly, the present invention provides a double-stranded oligo RNA structure in which a ligand (L), particularly a ligand having the property of binding specifically to a receptor that enhances target cell internalization by receptor-mediated endocytosis (RME), is further bound to the structure represented by any one of Structural Formulas (1) to (4), (7) and (8). For example, a structure in which a ligand is bound to the double-stranded oligo RNA structure represented by Structural Formula (1) has a structure shown in the following Structural Formula (15):

[Structural Formula (15)] (Lᵢ -Z)-A-X-R-Y-B

wherein A, B, X and Y are as defined in Structural Formula (1) above, L is a ligand having the property of binding specifically to a receptor that enhances target cell internalization by receptor-mediated endocytosis (RME), and "i" is an integer ranging from 1 to 5, preferably from 1 to 3.

The ligand in Structural Formula (15) may preferably be selected from among: target receptor-specific antibodies, aptamers and peptides, which have the RME property of enhancing target cell internalization; folate (the term "folate" is generally used interchangeably with folic acid, and the term "folate" as used herein means folate that is in a natural form or is activated in the human body); and chemical compounds, including hexosamines such as N-acetyl galactosamine (NAG), and sugars or carbohydrates such as glucose and mannose, without being limited thereto.

In addition, the hydrophilic compound (A) in Structural Formula (15) above may be used in the form of the hydrophilic block represented by Structural Formula (5) or (6).

In still another aspect, the present invention provides a method for producing a double-stranded oligonucleotide structure comprising an amphiregulin-specific double-stranded oligonucleotide.

For example, the method for producing a double-stranded oligonucleotide structure comprising an amphiregulin-specific double-stranded oligonucleotide according to the present invention may comprise steps of:
(1) binding a hydrophilic compound to a solid support;
(2) synthesizing an oligonucleotide single strand on the hydrophilic compound-bound solid support;
(3) covalently binding a hydrophobic compound to the 5' end of the oligonucleotide single strand;
(4) synthesizing an oligonucleotide single strand having a sequence complementary to the sequence of the oligonucleotide single strand of step (2);
(5) separating and purifying an oligonucleotide-polymer structure and the oligonucleotide single strand from the solid support after completion of synthesis; and
(6) annealing the produced oligonucleotide-polymer structure with the oligonucleotide single strand having the complementary sequence, thereby producing a double-stranded oligonucleotide structure.

The solid support that is used in the present invention is preferably controlled pore glass (CPG), without being limited thereto, and polystyrene (PS), polymethylmethacrylate (PMMA), silica gel, cellulose paper or the like may also be used. When CPG is used, it preferably has a diameter of 40 to 180 mm and a pore size of 500 to 3,000 Å. After step (5), the molecular weights of the produced and purified RNA-polymer structure and oligonucleotide single strand may be measured using a MALDI-TOF mass spectrometer in order to confirm that the desired oligonucleotide-polymer structure and oligonucleotide single strand were produced. In the above-described production method, step (4) of synthesizing the oligonucleotide single strand having a sequence complementary to the sequence of the oligonucleotide single strand synthesized in step (2) may be performed before step (1) or during any one step of steps (1) to (5).

In addition, the oligonucleotide single strand having a sequence complementary to the sequence of the oligonucleotide single strand synthesized in step (2) may be used in the state in which a phosphate group is bound to the 5' end of the oligonucleotide single strand.

Meanwhile, the present invention provides a method for producing a double-stranded oligonucleotide structure wherein a ligand is further bound to the double-stranded oligonucleotide structure comprising the amphiregulin-specific double-stranded oligonucleotide.

For example, the method for producing the ligand-bound double-stranded oligonucleotide structure comprising the amphiregulin-specific double-stranded oligonucleotide may comprise steps of:
(1) binding a hydrophilic compound to a solid support having a functional group bound thereto;
(2) synthesizing an oligonucleotide single strand on the solid support having the functional group and hydrophilic compound bound thereto;
(3) covalently binding a hydrophobic compound to the 5' end of the oligonucleotide single strand;
(4) synthesizing an oligonucleotide single strand having a sequence complementary to the sequence of the oligonucleotide single strand synthesized in step (2);
(5) separating the functional group-oligonucleotide-polymer structure and the oligonucleotide single strand having the complementary sequence from the solid support after completion of synthesis;
(6) binding a ligand to the end of the hydrophilic compound by the functional group to produce a ligand-oligonucleotide polymer structure single strand; and
(7) annealing the produced ligand-oligonucleotide-polymer structure with the oligonucleotide single strand having the complementary sequence, thereby producing a ligand/double-stranded-oligonucleotide structure.

After step (6), the produced ligand-oligonucleotide-polymer structure and the oligonucleotide single strand having the complementary sequence may be separated and purified, and then the molecular weights thereof may be measured using a MALDI-TOF mass spectrometer in order to confirm that the desired ligand-RNA-polymer structure and the desired RNA single strand having the complementary sequence were produced. By annealing the produced ligand/RNA-oligonucleotide structure with the oligonucleotide single strand having the complementary sequence, a ligand/double-stranded-oligonucleotide structure may be produced. In the above-described production method, step (4) of synthesizing the oligonucleotide single strand having a sequence complementary to the sequence of the oligonucleotide single strand synthesized in step (3) may be performed before step (1) or during any one step of steps (1) to (6)

In yet another aspect, the present invention is directed to nanoparticles comprising the double-stranded oligonucleotide structure according to the present invention. The double-stranded oligonucleotide according to the present invention forms self-assembled nanoparticles through hydrophobic interaction of the hydrophobic compound (Korean Patent No. 1224828). These nanoparticles have excellent *in vivo* delivery efficiency and *in vivo* stability. In addition, the high particle size uniformity of the nanoparticles makes quality control (QC) easy, and thus a process of preparing these nanoparticles as a drug is easy.

In the present invention, the nanoparticle may also be composed of a mixture of double-stranded oligonucleotide structures comprising double-stranded structures comprising different sequences. For example, the nanoparticle may comprise one kind of amphiregulin-specific double-stranded oligonucleotide comprising a sense strand, which comprises any one sequence selected from among SEQ ID NOs: 10 to 12, and an antisense strand comprising a sequence complementary thereto; however, in another embodiment, the nanoparticle may comprise different kinds of amphiregulin-specific double-stranded oligonucleotides, each comprising a sense strand, which comprises any one sequence selected from among SEQ ID NOs: 10 to 12, and an antisense strand comprising a sequence complementary thereto, and may also comprise an amphiregulin-specific double-stranded oligonucleotide which is not disclosed in the present invention.

For administration, the composition of the present invention may further comprise one or more pharmaceutically acceptable carriers, in addition to the above-described active ingredient. The pharmaceutically acceptable carriers should be compatible with the active ingredient, and may be selected from among physiological saline, sterile water, Ringer's solution, buffered saline, dextrose solution, maltodextrin solution, glycerol, ethanol, and a mixture of two or more thereof. If necessary, the composition may comprise other conventional additives such as an antioxidant, a buffer or a bacteriostatic agent. In addition, a diluent, a dispersing agent, a surfactant, a binder and a lubricant may additionally be added to the composition to prepare injectable formulations such as an aqueous solution, a suspension, and an emulsion. In particular, the composition is preferably provided as a lyophilized formulation. For the preparation of a lyophilized formulation, a conventional method known in the art to which the present invention pertains may be used, and a stabilizer for lyophilization may also be added. Furthermore, the composition may preferably be formulated depending on each disease or component by a suitable method known in the art or by a method disclosed in Remington's Pharmaceutical Science, Mack Publishing Company, Easton PA.

The dose of the composition of the present invention may be determined by a person skilled in the art based on the condition of the patient and the severity of the disease. In addition, the composition may be formulated in various dosage forms, including powders, tablets, capsules, liquids, injectable solutions, ointments and syrup formulations, and may be provided in unit-dosage or multi-dosage containers, for example, sealed ampules or vials.

The composition of the present invention may be administered orally or parenterally. The composition according to the present invention may be administered, for example, orally, via inhalation, intravenously, intramuscularly, intraarterially, intramedullary, intradurally, intracardially, transdermally, subcutaneously, intraperitoneally, intrarectally, sublingually, or topically, without being limited thereto. The dose of the composition according to the present invention may vary depending on the patient's weight, age, sex, health condition and diet, the duration of administration, the mode of administration, excretion rate, severity of disease, or the like, and may be easily determined by those skilled in the art. In addition, for clinical administration, the composition of the present invention may be prepared into a suitable formulation using a known technique.

In still yet another aspect, the present invention is directed to a lyophilized formulation comprising the pharmaceutical composition.

In a further aspect, the present invention is directed to a method of preventing or treating obesity comprising a step of administering, to a subject in need of prevention or treatment of obesity, any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] **A-X-R-Y-B**

   wherein A represents a hydrophilic compound, B represents a hydrophobic compound, X and Y each independently represent a simple covalent bond or a linkermediated covalent bond, and R represents the amphiregulin-specific double-stranded oligonucleotide (i); and
(iii) nanoparticles comprising the amphiregulin-specific double-stranded oligonucleotide structure (ii).

In another further aspect, the present invention is directed to a pharmaceutical composition for use in a method for preventing or treating obesity, the pharmaceutical composition comprising any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to the sense strand;
(ii) an amphiregulin-specific double-stranded oligonucleotide structure comprising a structure represented by the following Structural Formula (1):

   [Structural Formula (1)] **A-X-R-Y-B**

   wherein A represents a hydrophilic compound, B represents a hydrophobic compound, X and Y each independently represent a simple covalent bond or a linkermediated covalent bond, and R represents the amphiregulin-specific double-stranded oligonucleotide (i); and
(iii) nanoparticles comprising the amphiregulin-specific double-stranded oligonucleotide structure (ii).

In still another further aspect, the present invention is directed to the use of any one selected from the group consisting of:
(i) an amphiregulin-specific double-stranded oligonucleotide comprising a sense strand, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):

   [Structural Formula (1)] **A-X-R-Y-B**

   wherein A represents a hydrophilic compound, B represents a hydrophobic compound, X and Y each independently represent a simple covalent bond or a linkermediated covalent bond, and R represents the amphiregulin-specific double-stranded oligonucleotide (i); and
(iii) nanoparticles comprising the amphiregulin-specific double-stranded oligonucleotide structure (ii), in manufacture of a medicament for preventing or treating obesity.

In the present invention, the obesity may be visceral fat-type obesity caused by diabetes, without being limited thereto.

In the present invention, the amphiregulin-specific double-stranded oligonucleotide structure according to the present invention may exhibiting one or more of the following effects, without being limited to:
(i) loss of body weight,
(ii) reduction of food efficiency ratio,
(iii) reduction of subcutaneous fat,
(iv) reduction of visceral fat,
(v) reduction of adipocyte area, and
(vi) inhibition of liver adipogenesis.

In the present invention, the effect may be exhibited by inhibiting amphiregulin expression in adipose tissue, but the mechanism by which the effect is exhibited is not limited thereto.

Hereinafter, the present invention will be described in more detail with reference to examples. These examples are only for illustrating the present invention, and it will be obvious to those skilled in the art that the scope of the present invention is not to be construed as being limited by these examples.

In the present invention, three specific sequences capable of inhibiting amphiregulin expression were identified, and it was confirmed that these sequences could bind complementarily to an mRNA encoding amphiregulin and effectively inhibit amphiregulin expression, thereby effectively treating obesity-related diseases.

### Example 1. Algorithm for Screening of SAMiRNAs Targeting Amphiregulin and Selection of Candidate Sequences

SAMiRNA-based drug high-throughput screening is a method in which all possible candidate sequences are generated by applying a 1-base or 2-base sliding window algorithm to the entire mRNA, unnecessary candidate sequences are removed by performing homology filtering, and the degrees to which the expression of the gene of interest is inhibited by all the finally selected SAMiRNAs are determined.

First, a design process for SAMiRNA candidate sequences against amphiregulin was performed. Specifically, 1,257 SAMiRNA candidate sequences, each consisting of 19 nucleotides, were selected by applying a 1-base sliding window algorithm to the human amphiregulin mRNA NM_001657.3 (1,290 bp), and an experiment on the degree of inhibition of amphiregulin was performed.

### Example 2. Synthesis of Double-Stranded Oligo RNA Structure

A double-stranded oligo RNA structure (SAMiRNA) produced in the present invention is represented by the following structural formula:
C₂₄-5' S 3'-(hexaethyleneglycol-PO₃⁻)₃-hexaethyleneglycol AS 5'-PO₄

For synthesis of the sense strain of a monoSAMiRNA (n=4) double-stranded oligo structure, 3,4,6-triacetyl-1-hexa(ethylene glycol)-N-acetyl galactosamine-CPG was used as a support, and three demethoxytrityl (DMT) hexaethylene glycol phosphoramidates as hydrophilic monomers were continuously bound to the support through a reaction. Next, synthesis of RNA or DNA was performed, and then hydrophobic C₂₄ (C₆-S-S-C₁₈) containing a disulfide bond was bound to the 5' end region, thereby synthesizing the sense strand of monoSAMiRNA (n=4) in which NAG-hexaethyleneglycol-(-PO₃⁻ hexaethyleneglycol)₃ is bound to the 3' end and C₂₄ (C₆-S-S-C₁₈) is bound to the 5' end.

After completion of the synthesis, the synthesized RNA single strand and oligo (DNA or RNA)-polymer structure were detached from the CPG by treatment with 28%(v/v) ammonia in a water bath at 60°C, and then protective residues were removed by a deprotection reaction. After removal of the protective residues, the RNA single strand and the oligo (DNA or RNA)-polymer structure were treated with N-methylpyrrolidone, trimethylamine and triethylaminetrihydrofluoride at a volume ratio of 10:3:4 in an oven at 70°C to remove 2'-TBDMS (tert-butyldimethylsilyl). An RNA single strand, an oligo (DNA or RNA)-polymer structure and a ligand-bound oligo (DNA or RNA)-polymer structure were separated from the reaction products by high-performance liquid chromatography (HPLC), and the molecular weights thereof were measured using a MALDI-TOF mass spectrophotometer (MALDI TOF-MS, SHIMADZU, Japan) to confirm whether they would match the nucleotide sequence and polymer structure desired to be synthesized. Thereafter, to produce each double-stranded oligo structure, the sense strand and the antisense strand were mixed together, added to 1X annealing buffer (30 mM HEPES, 100 mM potassium acetate, 2 mM magnesium acetate, pH 7.0 to 7.5), allowed to react in a water bath at 90°C for 3 minutes, and then allowed to react at 37°C, thereby producing the desired SAMiRNA. Annealing of the produced double-stranded oligo RNA structures was confirmed by electrophoresis.

### Example 3. High-Throughput Screening (HTS) of SAMiRNA Nanoparticles That Target Human Amphiregulin and Induce RNAi

### 3-1 Production of SAMiRNA Nanoparticles

1,257 SAMiRNAs targeting amphiregulin sequences, synthesized in Example 2, were dissolved in 1X Dulbecco's phosphate buffered saline (DPBS) (WELGENE, KR) and freeze-dried in a freeze dryer (LGJ-100F, CN) for 5 days. The freeze-dried nanoparticle powders were dissolved and homogenized in 1.429 ml of deionized distilled water (Bioneer, KR) and used in an experiment for the present invention.

### 3-2 Treatment of Cells with SAMiRNA Nanoparticles

To identify SAMiRNA that inhibits amphiregulin expression, the human lung cancer line A549 was used. The A549 cell line was cultured in Gibco^{™} Ham's F-12K (Kaighn's) medium (Thermo, US) containing 10% fetal bovine serum (Hyclone, US) and 1% penicillin-streptomycin (Hyclone, US) at 37°C under 5% CO₂. Using the same medium as above, the A549 cell line was seeded in a 96-well plate (Costar, US) at a density of 2 × 10⁴ cells/well. The next day, the SAMiRNA homogenized with deionized distilled water in Example 3.1 above was diluted with 1X DPBS, and the cells were treated with the dilution at a SAMiRNA concentration of 500 nM or 1,000 nM. Treatment with the SAMiRNA was performed a total of four times (once every 12 hours), and the cells were cultured at 37°C under 5% CO₂.

### 3-3 Screening of SAMiRNA by Analysis of Inhibition of Human Amphiregulin mRNA Expression

Total RNA was extracted from the cell line treated with SAMiRNA in Example 3-2, and was synthesized into cDNA, and then the relative mRNA expression level of the amphiregulin gene was quantified by real-time PCR. For analysis of the mRNA expression level of the amphiregulin gene, 300 nM AREG forward primer, 300 nM AREG reverse primer, 300 nM AREG probe, 300 nM RPL13A forward primer, 300 nM RPL13A reverse primer, 300 nM RPL13A probe, 400 nM TBP forward primer, 400 nM TBP reverse primer, and 300 nM TBP probe were added to each well of an AccuPower^{®} Dual-HotStart RT-qPCR kit (Bioneer, Korea) and dried (Table 2 below shows the sequences of the primers and hydrolysis probes used in the high-throughput screening (HTS) experiment). To evaluate the performance of the prepared kit, a calibration curve was created using the A549 cell total RNA and the PCR amplification efficiency was determined (Table 3). RT-qPCR was performed under the following conditions: 95°C for 5 min, and then 45 cycles, each consisting of 95°C for 5 sec and 58°C for 15 sec. A protocol in which a fluorescence value is detected in each cycle was used.

The 96-well plate (Costar, US) treated with SAMiRNA was subjected to total RNA extraction, and onestep RT-qPCR was performed according to an automated program using the automated system ExiStation HT^{™} Korea and the separately prepared AccuPower^{®} Dual-HotStart RT-qPCR kit (Bioneer, Korea) comprising primers and probes for analysis of amphiregulin.

Based on the Ct values of two genes obtained after qPCR array, the relative mRNA expression level of amphiregulin in the test group compared to that in the control group was analyzed by the 2(-Delta Delta C(T)) method [Livak KJ, Schmittgen TD. 2001. Analysis of relative gene expression data using real-time quantitative PCR and the 2(-Delta Delta C(T)) Method. Methods. Dec; 25(4):4 02-8].

**[Table 2] Sequences of primers and hydrolysis probes used in high-throughput screening (HTS) experiment**

| | |
|---|---|
| AREG forward primer | CAGTGCTGATGGATTTGAGGT (SEQ ID NO: 26) |
| AREG reverse primer | ATAGCCAGGTATTTGTGGTTCG (SEQ ID NO: 27) |
| AREG probe | 5'FAM - TGAACCGTCCTCGGGAGCCGACT - 3'EBQ (SEQ ID NO: 28) |
| RPL13A forward primer | GTGTTTGACGGCATCCCACC (SEQ ID NO: 29) |
| RPL13A reverse primer | TAGGCTTCAGACGCACGACC (SEQ ID NO: 30) |
| RPL13A probe | 5'TAMRA- AAGCGGATGGTGGTTCCTGCT - 3'EBQ (SEQ ID NO: 31) |
| TBP forward primer | CACCACAGCTCTTCCACTC (SEQ ID NO: 32) |
| TBP reverse primer | ATCCCAGAACTCTCCGAAGC (SEQ ID NO: 33) |
| TBP probe | 5'TEXASRED - ACCCTTGCCGGGCACCACTC - 3'EBQ (SEQ ID NO: 34) |

**[Table 3] 3-plex RT-qPCR amplification efficacy**

| | Slope | R² | Efficiency |
|---|---|---|---|
| AREG | Y=-0.2778X+12.3894 | 0.9998 | 900 |
| RPL13A | Y=-0.2863X+10.5964 | 0.9999 | 930 |
| TBP | Y=-0.2892X+13.0351 | 0.9946 | 950 |

To select highly efficient SAMiRNA, 14 SAMiRNAs were selected, which had each of the sequences of SEQ ID NOs: 1 to 14 as a sense strand. Here, the selected SAMiRNAs showed the highest efficiency with which the mRNA expression level of amphiregulin at a final concentration of 500 nM or 1,000 nM decreased compared to the control.

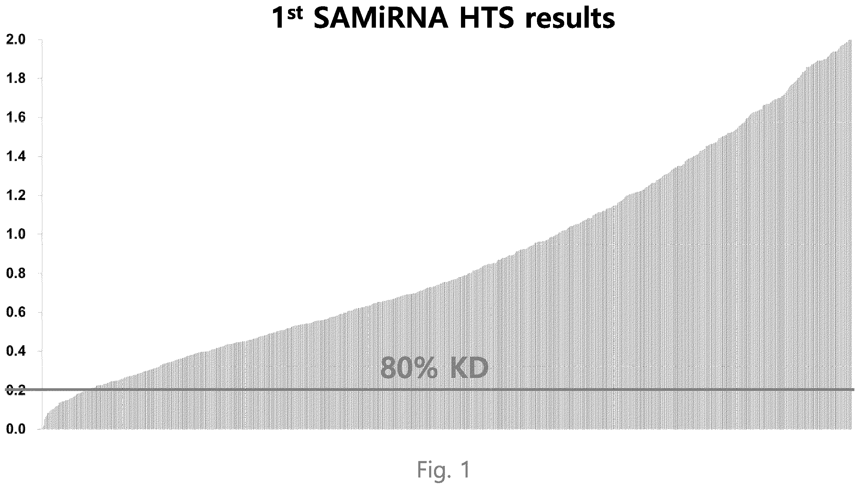

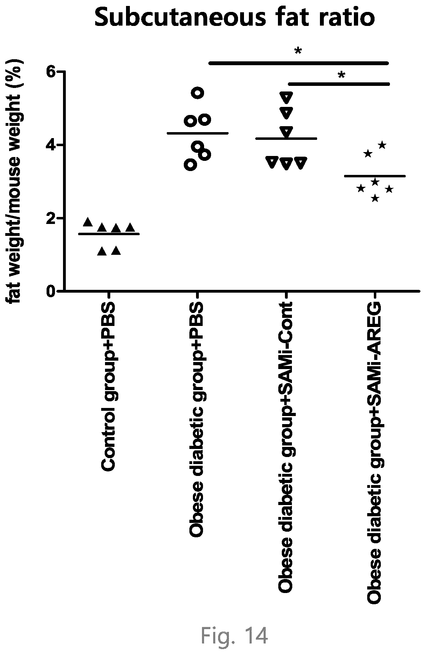

As shown in FIG. 1, 14 SAMiRNAs that most effectively inhibit amphiregulin gene expression were finally selected from 1,257 SAMiRNAs targeting amphiregulin. Information on the sequences of the selected SAMiRNAs is shown in Table 4 below.

**[Table 4] Amphiregulin-specific SAMiRNA candidate sequences selected by 1-base sliding window screening and high-throughput screening (HTS)**

| **SEQ ID NO** | **Accession No.** | **Position** | **Sequence (DNA/RNA)** | |
|---|---|---|---|---|
| 1 | NM_001657.3 | 8-26 | **Sense** | CCTATAAAGCGGCAGGTGC |
| 35 | | | **Antisense** | GCACCUGCCGCUUUAUAGG |
| 2 | **NM_001657.3** | 130-148 | **Sense** | GAGCGGCGCACACTCCCGG |
| 36 | | | **Antisense** | CCGGGAGUGUGCGCCG CUC |
| 3 | NM_001657.3 | 195-213 | **Sense** | GTCCCAGAGACCGAGTTGC |
| 37 | | | **Antisense** | GCAACUCGGUCUCUGG GAC |
| 4 | **NM_001657.3** | 224-242 | **Sense** | GAGACGCCGCCGCTGCGAA |
| 38 | | | **Antisense** | UUCGCAGCGGCGGCGU CUC |
| 5 | NM_001657.3 | 270-288 | **Sense** | CCGGCGCCGGTGGTGCTGT |
| 39 | | | **Antisense** | ACAGCACCACCGGCGC CGG |
| 6 | NM_001657.3 | 278-296 | **Sense** | GGTGGTGCTGTCGCTCTTG |
| 40 | | | **Antisense** | CAAGAGCGACAGCACC ACC |
| 7 | NM_001657.3 | 289-307 | **Sense** | CGCTCTTGATACTCGGCTC |
| 41 | | | **Antisense** | GAGCCGAGUAUCAAGA GCG |
| 8 | NM_001657.3 | 292-310 | **Sense** | TCTTGATACTCGGCTCAGG |
| 42 | | | **Antisense** | CCUGAGCCGAGUAUCA AGA |
| 9 | NM_001657.3 | 329-347 | **Sense** | GGACCTCAATGACACCTAC |
| 43 | | | **Antisense** | GUAGGUGUCAUUGAGG UCC |
| 10 | NM_001657.3 | 341-359 | **Sense** | CACCTACTCTGGGAAGCGT |
| 44 | | | **Antisense** | ACGCUUCCCAGAGUAG GUG |
| 11 | NM_001657.3 | 342-360 | **Sense** | ACCTACTCTGGGAAGCGTG |
| 45 | | | **Antisense** | CACGCUUCCCAGAGUA GGU |
| 12 | NM_001657.3 | 349-367 | **Sense** | CTGGGAAGCGTGAACCATT |
| 46 | | | **Antisense** | AAUGGUUCACGCUUCC CAG |
| 13 | NM_001657.3 | 353-371 | **Sense** | GAAGCGTGAACCATTTTCT |
| 47 | | | **Antisense** | AGAAAAUGGUUCACGC UUC |
| 14 | NM_001657.3 | 368-386 | **Sense** | TTCTGGGGACCACAGTGCT |
| 48 | | | **Antisense** | AGCACUGUGGUCCCCA GAA |

### Example 4. Screening of SAMiRNA Nanoparticles That Target Human Amphiregulin and Induce RNAi

The lung cancer cell line A549 was treated with SAMiRNA (selected in Example 3) having each of the sequences of SEQ ID NOs: 1 to 14 as a sense strand, and the expression pattern of amphiregulin mRNA in the cell line was analyzed.

### 4-1 Treatment of Cells with SAMiRNA Nanoparticles

To identify SAMiRNA that inhibits amphiregulin expression, the human lung cancer line A549 was used. The A549 cell line was cultured in Gibco^{™} Ham's F-12K (Kaighn's) medium (Thermo, US) containing 10% fetal bovine serum (Hyclone, US) and 1% penicillin-streptomycin (Hyclone, US) at 37°C under 5% CO₂. Using the same medium as above, the A549 cell line was seeded in a 12-well plate (Costar, US) at a density of 8 × 10⁴ cells/well. The next day, the SAMiRNA homogenized with deionized distilled water in Example 3.1 above was diluted with 1X DPBS, and the cells were treated with the dilution to a SAMiRNA concentration of 200 nM or 600 nM. Treatment with the SAMiRNA was performed a total of four times (once every 12 hours), and the cells were cultured at 37°C under 5% CO₂.

### 4-2 Screening of SAMiRNA by Analysis of Inhibition of Human Amphiregulin mRNA Expression

Total RNA was extracted from the cell line treated with SAMiRNA in Example 4-1 and was synthesized into cDNA, and then the relative mRNA expression level of the amphiregulin gene was quantified by real-time PCR.

### 4-2-1 RNA Isolation from SAMiRNA-Treated Cells and cDNA Synthesis

Using an RNA extraction kit (AccuPrep Cell total RNA extraction kit, BIONEER, Korea), total RNA was extracted from the cell line treated with SAMiRNA in Example 4-1 above. The extracted RNA was synthesized into cDNA in the following manner using RNA reverse transcriptase (AccuPower^{®} RocketScript^{™}Cycle RT Premix with oligo (dT)20, Bioneer, Korea). Specifically, 1 µg of the extracted RNA was added to AccuPower^{®} RocketScript^{™}Cycle RT Premix with oligo (dT)20 (Bioneer, Korea) in each 0.25 ml Eppendorf tube, and distilled water treated with DEPC (diethyl pyrocarbonate) was added thereto to a total volume of 20 µL. In a gene amplification system (MyGenie^{™}96 Gradient Thermal Block, BIONEER, Korea), a process of hybridizing the RNA with primers at 37°C for 30 seconds and a process of synthesizing cDNA at 48°C for 4 minutes were repeated 12 times. Then, the amplification reaction was terminated by deactivating the enzyme at 95°C for 5 minutes.

### 4-2-2 Quantitative Analysis of Relative mRNA Expression Level of Human Amphiregulin mRNA

Using the cDNA synthesized in Example 4-2-1 as a template, SYBR green real-time qPCR was performed, and the relative mRNA expression level of amphiregulin compared to a SAMiRNA control sample was analyzed in the following manner. The cDNA synthesized in Example 4-2-1 above was diluted 5-fold with distilled water, and for analysis of the mRNA expression level of amphiregulin, 3 µl of the diluted cDNA, 25 µl of AccuPower^{®} 2X GreenStar^{™} qPCR MasterMix (BIONEER, Korea), 19 µl of distilled water, and 3 µl of amphiregulin qPCR primers (SEQ ID NOs: 17 and 18 (Table 5); 10 pmole/µl of each primer, BIONEER, Korea) were added to each well of a 96-well plate to prepare a mixture. Meanwhile, GAPDH (glyceraldehyde 3-phosphate dehydrogenase), a housekeeping gene (hereinafter referred to as HK gene), was used as a standard gene to normalize the mRNA expression level of amphiregulin. The 96-well plate containing the mixture was subjected to the following reaction using Exicycler^{™} Real-Time Quantitative Thermal Block (BIONEER, Korea). Specifically, the mixture was allowed to react at 95°C for 15 minutes to activate the enzyme and remove the secondary structure of the cDNA, and then the mixture was subjected to 42 cycles, each consisting of denaturation at 94°C for 30 sec, annealing at 58°C for 30 sec, extension at 72°C for 30 sec, and SYBR green scan, and to final extension at 72°C for 3 minutes. Next, the mixture was maintained at a temperature of 55°C for 1 minute, and the melting curve from 55°C to 95°C was analyzed.

After completion of the PCR, the Ct (threshold cycle) value of the target gene was corrected by the GAPDH gene, and then the ΔCt value was calculated using a control treated with the control sequence SAMiRNA (SAMiCONT) that does not induce gene expression inhibition. The relative expression level of the target gene in the cells treated with the amphiregulin-specific SAMiRNA was quantified using the ΔCt value and the equation 2(-ΔCt)×100.

To select highly efficient SAMiRNAs, three SAMiRNAs were selected, which had each of the sequences of SEQ ID NOs: 10, 11 and 12 as a sense strand. Here, the selected SAMiRNAs showed the highest efficiency with which the mRNA expression level of amphiregulin at a final concentration of 200 nM or 600 nM decreased compared to the control.

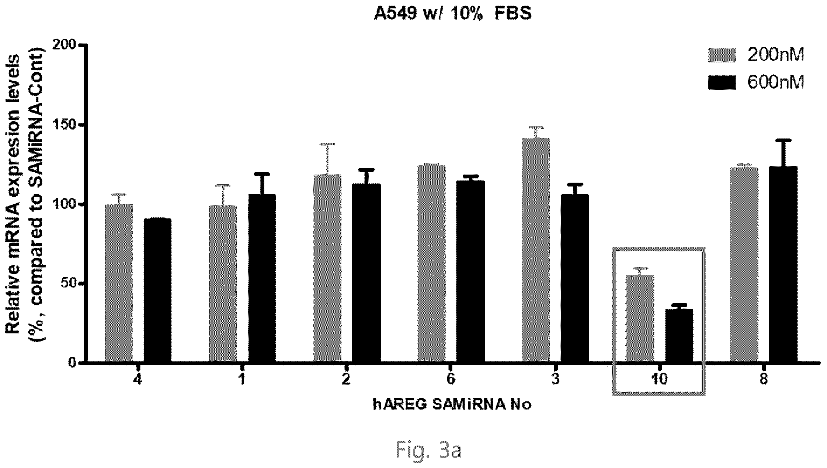

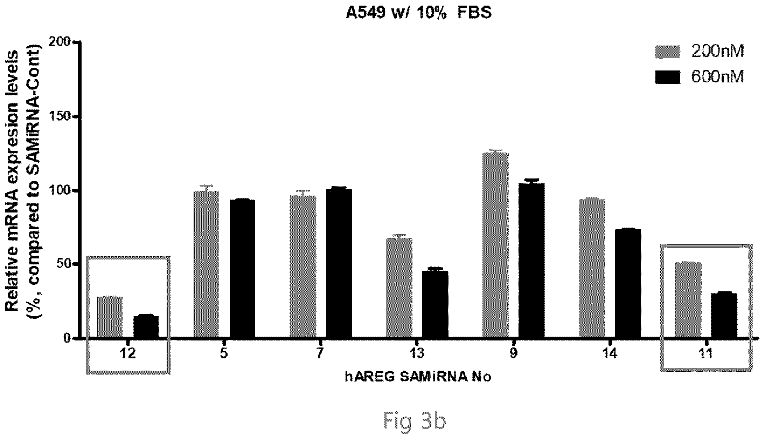

As shown in FIG. 3, three SAMiRNAs that most effectively inhibit amphiregulin gene expression were finally selected from 14 SAMiRNAs targeting amphiregulin. Information on the sequences of the selected SAMiRNAs is shown in Table 6 below.

**[Table 5] Information on primer sequences for qPCR**

| Primer | Sequence | SEQ ID NO |
|---|---|---|
| hGAPDH-F | GGTGAAGGTCGGAGTCAACG | 15 |
| hGAPDH-R | ACCATGTAGTTGAGGTCAATGAAGG | 16 |
| hAREG-F | ACACCTACTCTGGGAAGCGT | 17 |
| hAREG-R | GCCAGGTATTTGTGGTTCGT | 18 |

| | | |
|---|---|---|
| (F denotes a forward primer, and R denotes a reverse primer) | | |

**[Table 6] SAMiRNA sequences that effectively inhibit amphiregulin expression**

| SEQ ID NO | Code Name | Position | Sense strand sequence |
|---|---|---|---|
| 10 | SAMi-AREG#10 | 341-359 | CACCTACTCTGGGAAGCGT |
| 11 | SAMi-AREG#11 | 342-360 | ACCTACTCTGGGAAGCGTG |
| 12 | SAMi-AREG#12 | 349-367 | CTGGGAAGCGTGAACCATT |

### Example 5. Inhibition of Human Amphiregulin Expression in Lung Cancer Cell Line (A549) by Selected SAMiRNAs

The lung cancer cell line A549 was treated with the SAMiRNA (selected in Example 4) having each of the sequences of SEQ ID NOs: 10, 11 and 12 as a sense strand, and the expression pattern of amphiregulin mRNA in the cell line was analyzed to determine the IC₅₀ value of the SAMiRNA.

### 5-1 Production and Particle Size Analysis of SAMiRNA Nanoparticles

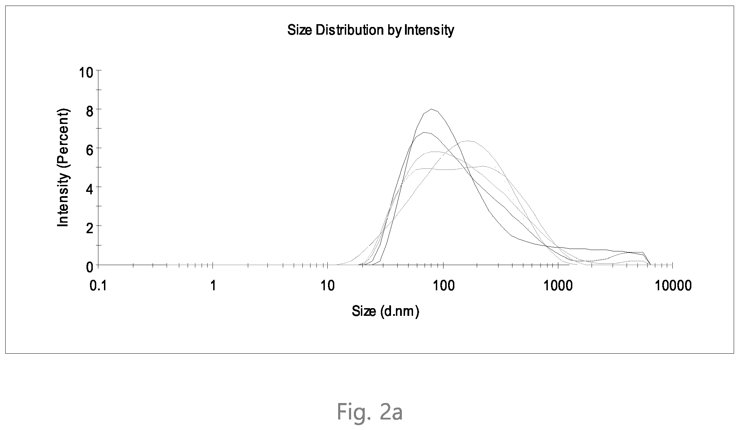

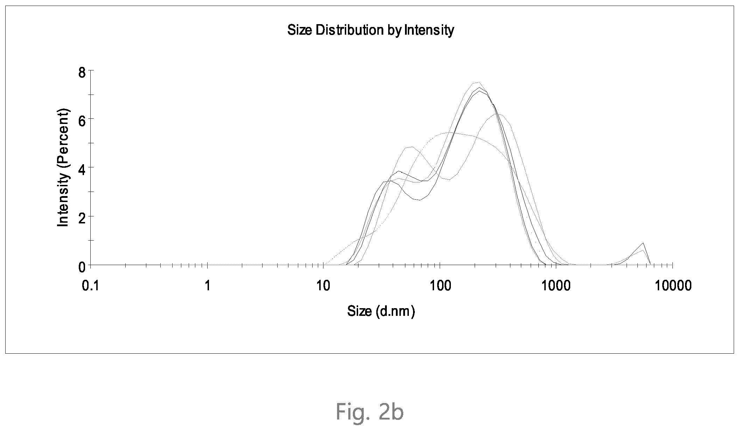

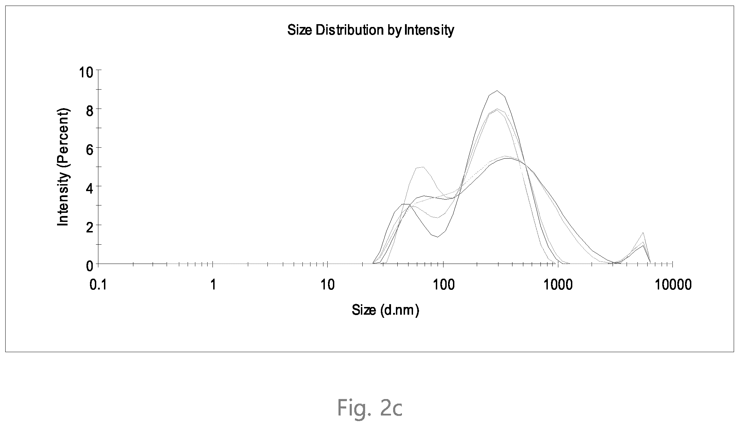

Each of the three SAMiRNAs targeting the amphiregulin sequence, synthesized in Example 2, was dissolved in 1X Dulbecco's phosphate buffered saline (DPBS) (WELGENE, KR) and freeze-dried in a freeze dryer (LGJ-100F, CN) for 5 days. The freeze-dried nanoparticle powders were dissolved and homogenized in 2 ml of deionized distilled water (Bioneer, KR) and used in an experiment for the present invention. To analyze the particle size of the produced SAMiRNA nanoparticles, the size and polydispersity index of the SAMiRNA were measured using a Zetasizer Nano ZS (Malvern, UK). The results of measuring the size and polydispersity index of the SAMiRNA nanoparticles are shown in Table 7 below and graphically shown in FIG. 2.

**[Table 7] Size and polydispersity index of amphiregulin-specific SAMiRNA nanoparticles**

| **Code Name** | **Size** | **PDI** |
|---|---|---|
| **SAMi-AREG#10** | **103.9±3.8** | **0.406±0.065** |
| **sAMi-AREG#11** | **99.9±4.0** | **0.501±0.005** |
| **SAMi-AREG#12** | **170.1±7.5** | **0.457±0.084** |

### 5-2 Treatment of Cells with SAMiRNA Nanoparticles

To evaluate the effect of the selected SAMiRNAs that inhibit amphiregulin expression, the human lung cancer cell line A549 was used. The A549 cell line was cultured in Gibco^{™} Ham's F-12K (Kaighn's) medium (Thermo, US) containing 10% fetal bovine serum (Hyclone, US) and 1% penicillin-streptomycin (Hyclone, US) at 37°C under 5% CO₂. Using the same medium as above, the A549 cell line was seeded in a 12-well plate (Costar, US) at a density of (Costar, US) 8 × 10⁴ cells/well. The next day, the SAMiRNA homogenized with deionized distilled water in Example 5.1 above was diluted with 1X DPBS, and the cells were treated with the dilution to a SAMiRNA concentration of 12.5 nM, 25 nM, 50 nM, 100 nM, 200 nM, 600 nM or 1200 nM. Treatment of the cells with the SAMiRNA was performed a total of four times (once every 12 hours), and the cells were cultured at 37°C under 5% CO₂.

### 5-3 Determination of IC₅₀ of SAMiRNA by Analysis of Inhibition of Human Amphiregulin mRNA Expression

Total RNA was extracted from the cell line treated with the SAMiRNA in Example 5-2 and was synthesized into cDNA, and then the relative mRNA expression level of the amphiregulin gene was quantified by real-time PCR.

### 5-3-1 RNA Isolation from SAMiRNA-Treated Cells and cDNA Synthesis

Using an RNA extraction kit (AccuPrep Cell total RNA extraction kit, BIONEER, Korea), total RNA was extracted from the cell line treated with the SAMiRNA in Example 5-2 above. The extracted RNA was synthesized into cDNA in the following manner using RNA reverse transcriptase (AccuPower^{®} RocketScript^{™}Cycle RT Premix with oligo (dT)20, Bioneer, Korea). Specifically, 1 µg of the extracted RNA was added to AccuPower^{®} RocketScript^{™}Cycle RT Premix with oligo (dT)20 (Bioneer, Korea) in each 0.25 ml Eppendorf tube, and distilled water treated with DEPC (diethyl pyrocarbonate) was added thereto to a total volume of 20 µl. In a gene amplification system (MyGenie^{™}96 Gradient Thermal Block, BIONEER, Korea), a process of hybridizing the RNA with primers at 37°C for 30 seconds and a process of synthesizing cDNA at 48°C for 4 minutes were repeated 12 times. Then, the amplification reaction was terminated by deactivating the enzyme at 95°C for 5 minutes.

### 5-3-2 Quantitative Analysis of Relative mRNA Expression Level of Human Amphiregulin

Using the cDNA synthesized in Example 5-3-1 as a template, SYBR green real-time qPCR was performed, and the relative mRNA expression level of amphiregulin compared to a SAMiRNA control sample was analyzed in the following manner. The cDNA synthesized in Example 5-3-1 above was diluted 5-fold with distilled water, and for analysis of the mRNA expression level of amphiregulin, 3 µl of the diluted cDNA, 25 µl of AccuPower^{®} 2X GreenStar^{™} qPCR MasterMix (BIONEER, Korea), 19 µl of distilled water, and 3 µl of amphiregulin qPCR primers (SEQ ID NOs: 17 and 18 (Table 5); 10 pmole/µl for each primer, BIONEER, Korea) were added to each well of a 96-well plate to make a mixture. Meanwhile, GAPDH (glyceraldehyde 3-phosphate dehydrogenase), a housekeeping gene (hereinafter referred to as HK gene), was used as a standard gene to normalize the mRNA expression level of amphiregulin. The 96-well plate containing the mixture was subjected to the following reaction using an Exicycler^{™} Real-Time Quantitative Thermal Block (BIONEER, Korea). Specifically, the mixture was allowed to react at 95°C for 15 minutes to activate the enzyme and remove the secondary structure of the cDNA, and then the mixture was subjected to 42 cycles, each consisting of denaturation at 94°C for 30 sec, annealing at 58°C for 30 sec, extension at 72°C for 30 sec, and SYBR green scan, and to final extension at 72°C for 3 minutes. Next, the mixture was maintained at a temperature of 55°C for 1 minute, and the melting curve from 55°C to 95°C was analyzed.

After completion of the PCR, the Ct (threshold cycle) value of the target gene was corrected by the GAPDH gene, and then the ΔCt value was calculated using a control treated with the control sequence SAMiRNA (SAMiCONT) that does not induce gene expression inhibition. The relative expression level of the target gene in the cells treated with the amphiregulin-specific SAMiRNA was quantified using the ΔCt value and the equation 2(-ΔCt) × 100.

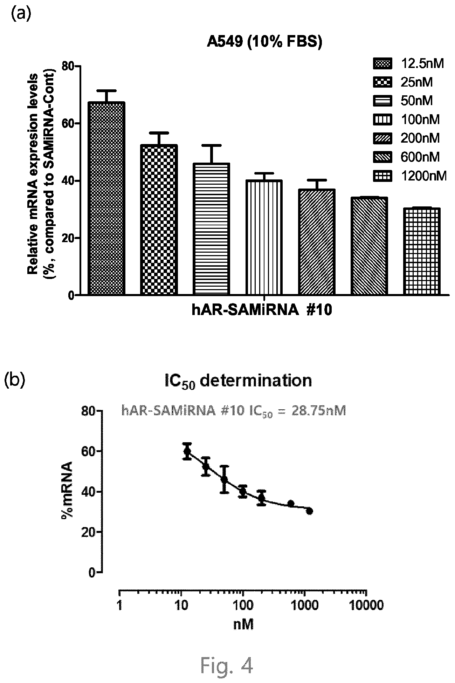

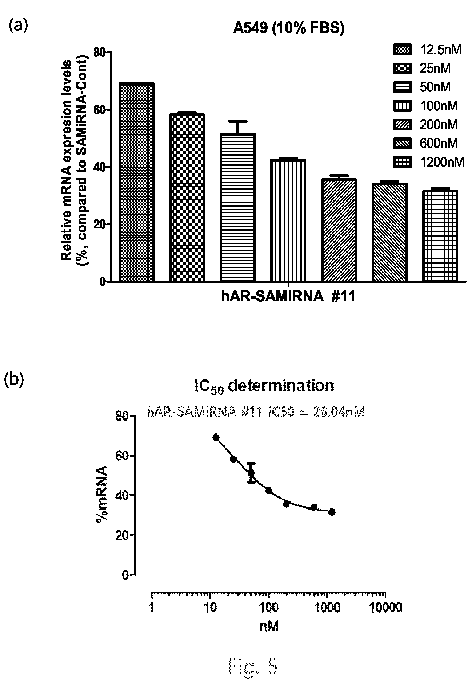

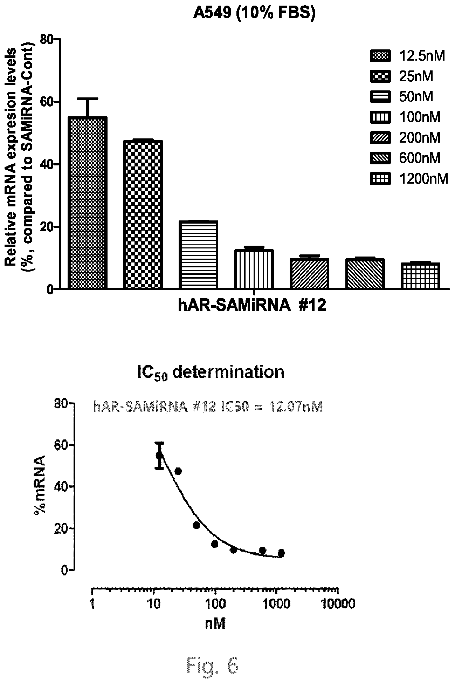

As a result, it was confirmed that all the amphiregulin-specific SAMiRNAs having each of the sequences of SEQ ID NOs: 10, 11 and 12 as a sense strand showed a 50% or more decrease in the mRNA expression level of amphiregulin even at a low concentration of 100 nM, suggesting that the amphiregulin-specific SAMiRNAs exhibited the effect of inhibiting amphiregulin expression with high efficiency. It was confirmed that the IC₅₀ values were 28.75 nM as shown in FIG. 4 for the amphiregulin-specific SAMiRNA having the sequence of SEQ ID NO: 10 as a sense strand, 26.04 nM as shown in FIG. 5 for the amphiregulin-specific SAMiRNA having the sequence of SEQ ID NO: 11 as a sense strand, and 12.07 nM as shown in FIG. 6 for the amphiregulin-specific SAMiRNA having the sequence of SEQ ID NO: 12 as a sense strand. In particular, it was confirmed that the amphiregulin-specific SAMiRNA having the sequence of SEQ ID NO: 12 as a sense strand showed a 50% or more decrease in the mRNA expression level of amphiregulin even at a low concentration of 25 nM as shown in FIG. 6, suggesting that it exhibited the effect of most effectively inhibiting amphiregulin gene expression among the three selected sequences.

### Example 6. Evaluation of In Vitro Cytotoxicity Using Human Peripheral Blood Mononuclear Cells (PBMCs)

In order to examine whether the mRNA expression levels of innate immune-related cytokines are increased by SAMi-hAREG, ePBMC^{®} cryopreserved human PBMCs (human peripheral monocular cells, Cellular Technology Limited, USA) were seeded at a density of 5 × 10⁵ cells per well in a 12-well plate (Costar^{®} USA) with RPMI1640 (Hyclone^{™}) medium containing 10% FBS (fetal bovine serum; Hyclone^{™}). The cells were cultured in a 5% CO₂ incubator at 37°C for 1 hour so as to be stabilized, and then the seeded PBMCs were treated with 2.5 µM of each of SAMiCON (DNA/RNA), SAMi-hAREG#10 (DNA/RNA), SAMi-hAREG#11 (DNA/RNA), SAMi-hAREG#12 (DNA/RNA), SAMiCON (RNA/RNA), SAMi-hAREG#10 (RNA/RNA), SAMi-hAREG#11 (RNA/RNA), and SAMi-hAREG#12 (RNA/RNA), and cultured in a 5% CO₂ incubator at 37°C for 6 hours. As a positive control, 20 µg/ml of Concanavalin A (Sigma Aldrich, USA) was used.

Thereafter, all the cells were harvested, and total RNA was extracted therefrom using an RNeasy Mini Kit (Qiagen, Germany) and an RNase-Free DNase Set (Qiagen, Germany) according to the manufacturer's protocols.

200 ng of the extracted RNA was mixed with deionized sterile DW (Bioneer, Korea) and RNA reverse transcriptase (AccuPower^{®} RocketScript^{™}Cycle RT Premix with oligo (dT)20, Bioneer, Korea), and the mixture was allowed to react using a gene amplification system (MyGenie^{™}96 Gradient Thermal Block, BIONEER, Korea) under conditions of 12 cycles, each consisting of 37°C for 30 sec, 48°C for 4 min and 55°C for 30 sec, and then 95°C for 5 min, thereby synthesizing a total of 20 µl of cDNA.

The synthesized cDNA was mixed with qPCR primers for each of RPL13A, IL1B, IL6, IFNG, TNF and IL12B genes and then amplified using an Exicycler^{™}96 Real-Time Quantitative Thermal Block (Bioneer, Korea) under the following conditions: 95°C for 5 min, and then 45 cycles, each consisting of 95°C for 5 sec and 58°C for 15 sec.

Based on the Ct values of two genes obtained after qPCR array, the relative mRNA expression level (%) in the test group compared to that in the control group was analyzed by the 2(-Delta Delta C(T)) Method [Livak KJ, Schmittgen TD. 2001. Analysis of relative gene expression data using real-time quantitative PCR and the 2(-Delta Delta C(T)) Method. Methods. Dec; 25(4):4 02-8].

As a result, as shown in FIG. 7, it was confirmed that the expression of innate immune-related cytokines in the human peripheral blood mononuclear cells (human PBMCs) by each of amphiregulin-specific SAMiRNA #10, SAMiRNA #11 and SAMiRNA #12 was not observed.

### Example 7. Comparative Analysis of the Inhibition of Human Amphiregulin Expression by DNA/RNA Hybrid and RNA/RNA Hybrid SAMiRNAs Comprising Each of Selected Sequences of SEQ ID NOs: 10, 11 and 12 as Sense Strand

The lung cancer cell line A549 was treated with each of a double stranded DNA/RNA hybrid and RNA/RNA hybrid comprising the amphiregulin-specific SAMiRNA (selected in Example 4) having each of the sequences of SEQ ID NOs: 10, 11 and 12 as a sense strand, and the relative mRNA expression levels (%) of amphiregulin in the cell line were comparatively analyzed.

### 7-1 Treatment of Cells with SAMiRNA Nanoparticles

To identify SAMiRNA that inhibits amphiregulin expression, the human lung cancer line A549 was used. The A549 cell line was cultured in Gibco^{™} Ham's F-12K (Kaighn's) medium (Thermo, US) containing 10% fetal bovine serum (Hyclone, US) and 1% penicillin-streptomycin (Hyclone, US) at 37°C under 5% CO₂. Using the same medium as above, the A549 cell line was seeded in a 12-well plate (Costar, US) at a density of 8 × 10⁴ cells/well. The next day, the SAMiRNA homogenized with deionized distilled water in Example 3.1 above was diluted with 1X DPBS, and the cells were treated with the dilution to a SAMiRNA concentration of 200 nM, 600 nM or 1200 nM. Treatment of the cells with the SAMiRNA was performed a total of four times (once every 12 hours), and the cells were cultured at 37°C under 5% CO₂.

### 7-2 Screening of SAMiRNA by Analysis of Inhibition of Human Amphiregulin mRNA Expression

Total RNA was extracted from the cell line treated with SAMiRNA in Example 7-1 and was synthesized into cDNA, and then the relative mRNA expression level of the amphiregulin gene was quantified by real-time PCR.

### 7-2-1 RNA Isolation from SAMiRNA-Treated Cells and cDNA Synthesis

Using an RNA extraction kit (AccuPrep Cell total RNA extraction kit, BIONEER, Korea), total RNA was extracted from the cell line treated with SAMiRNA in Example 7-1 above. The extracted RNA was synthesized into cDNA in the following manner using RNA reverse transcriptase (AccuPower^{®} RocketScript^{™}Cycle RT Premix with oligo (dT)20, Bioneer, Korea). Specifically, 1 µg of the extracted RNA was added to AccuPower^{®} RocketScript^{™}Cycle RT Premix with oligo (dT)20 (Bioneer, Korea) in each 0.25 µl Eppendorf tube, and distilled water treated with DEPC (diethyl pyrocarbonate) was added thereto to a total volume of 20 µl. In a gene amplification system (MyGenie^{™}96 Gradient Thermal Block, BIONEER, Korea), a process of hybridizing the RNA with primers at 37°C for 30 seconds and a process of synthesizing cDNA at 48°C for 4 minutes were repeated 12 times. Then, the amplification reaction was terminated by deactivating the enzyme at 95°C for 5 minutes.

### 7-2-2 Quantitative Analysis of Relative Human Amphiregulin mRNA Expression Level

Using the cDNA synthesized in Example 7-2-1 as a template, SYBR green real-time qPCR was performed, and the relative mRNA expression level of amphiregulin compared to a SAMiRNA control sample was analyzed in the following manner. The cDNA synthesized in Example 7-2-1 above was diluted 5-fold with distilled water, and for analysis of the mRNA expression level of amphiregulin, and 3 µl of the diluted cDNA, 25 µl of AccuPower^{®} 2X GreenStar^{™} qPCR MasterMix (BIONEER, Korea), 19 µl of distilled water, and 3 µl of amphiregulin qPCR primers (SEQ ID NOs: 17 and 18 (Table 5); 10 pmole/ml for each primer, BIONEER, Korea) were added to each well of a 96-well plate to prepare a mixture. Meanwhile, GAPDH (glyceraldehyde 3-phosphate dehydrogenase), a housekeeping gene (hereinafter referred to as HK gene), was used as a standard gene to normalize the mRNA expression level of amphiregulin. The 96-well plate containing the mixture was subjected to the following reaction using an Exicycler^{™} Real-Time Quantitative Thermal Block (BIONEER, Korea). Specifically, the mixture was allowed to react at 95°C for 15 minutes to activate the enzyme and remove the secondary structure of the cDNA, and then the mixture was subjected to 42 cycles, each consisting of denaturation at 94°C for 30 sec, annealing at 58°C for 30 sec, extension at 72°C for 30 sec, and SYBR green scan, and to final extension at 72°C for 3 minutes. Next, the mixture was maintained at a temperature of 55°C for 1 minute, and the melting curve from 55°C to 95°C was analyzed.

After completion of the PCR, the Ct (threshold cycle) value of the target gene was corrected by the GAPDH gene, and then the ΔCt value was calculated using a control treated with the control sequence SAMiRNA (SAMiCONT) that does not induce gene expression inhibition. The relative expression level of the target gene was quantified using the ΔCt value and the equation 2(-ΔCt)×100.

To select highly efficient SAMiRNA from the double-stranded DNA/RNA hybrid and RNA/RNA hybrids, the DNA/RNA hybrid SAMiRNA having the sequence of SEQ ID NO: 12 as a sense strand was finally selected. Here, the selected sequence DNA/RNA hybrid SAMiRNA (a gene expression inhibition of 90% or more) showed the highest efficiency with which the mRNA expression level of amphiregulin at a final concentration of 200 nM, 600 nM or 1200 nM decreased compared to the control.

As shown in FIG. 8, the DNA/RNA hybrid SAMiRNA 12 that most effectively inhibits amphiregulin gene expression was finally selected from the DNA/RNA and RNA/RNA hybrids comprising the three selected amphiregulin-specific SAMiRNAs, respectively.

### Example 8. High-Throughput Screening (HTS) of SAMiRNA Nanoparticles That Target Mouse Amphiregulin and Induce RNAi

In the case of siRNA therapeutic agents, it is difficult to identify an optimal sequence that is applicable to different strains. In this case, US FDA guidelines are applied, according to which a DNA sequence (surrogate sequence; mouse gene-specific siRNA) specific for an animal model for analysis of therapeutic effects (an *in vivo* efficacy test) is designed so as to verify pharmacological activity resulting from the inhibition of expression of the gene of interest and toxicity resulting from the inhibition of expression of the gene of interest (presentation by Robert T. Dorsam Ph.D. Pharmacology/Toxicology Reviewer, FDA/CDER).

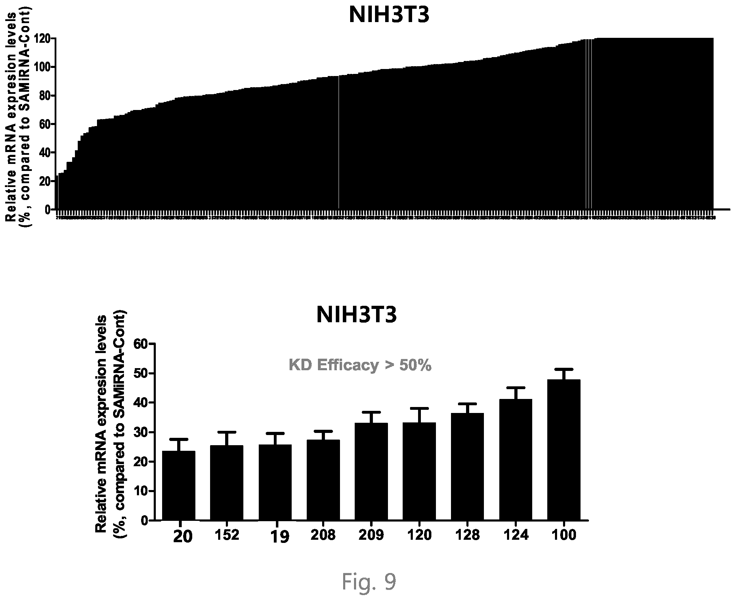

Previously discovered screening was modified by existing algorithm-based siRNA program (Turbo-si-designer owned by the applicant's company), and SAMiRNA-based siRNA sequence high-throughput screening was performed. 1-base sliding window scanning (the same method as the above-described human amphiregulin target screening) of 19-mer siRNAs against the entire target gene was performed, and a total of 1,190 candidate siRNA sequences against the possible mouse amphiregulin gene (NM_009704.4) full transcript sequence were generated. Blast sequence homology filtering was performed to remove unnecessary candidate sequences that influence the expression of other genes, and 237 finally selected SAMiRNAs were synthesized. The mouse NIH3T3 cell line was treated with each selected SAMiRNA at a concentration of 1 µM in a cell culture medium containing 10% FBS, and the *in vitro* expression inhibitory effects of the SAMiRNAs were first screened using the primers shown in Table 8 (primer sequence information for qPCR) (FIG. 9).

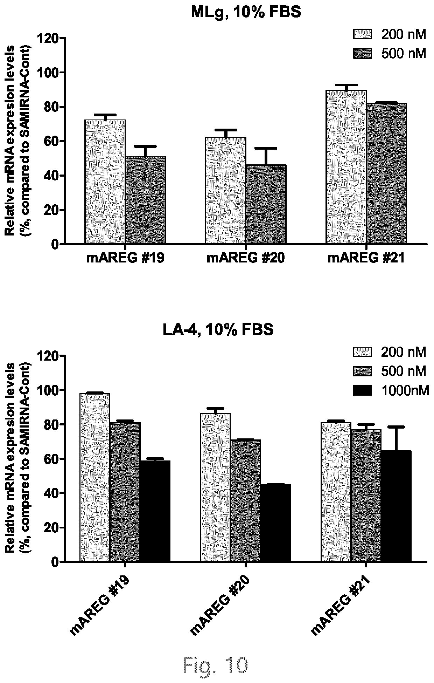

Thereafter, the mouse lung fibroblast cell line MLg was treated with each of the two sequences (SEQ ID NOs: 19 and 20) selected in the NIH3T3 cell line and the mouse SAMiRNA-amphiregulin of SEQ ID NO: 21 discovered through previous milestone studies, at treatment concentrations of 200 nM and 500 nM in cell culture media containing 10% FBS, and additional screening was performed. As a result, it was confirmed that SEQ ID NO: 20 exhibited the best expression inhibitory effect (FIG. 10a) .

Additionally, the mouse lung epithelial cell line LA-4 (ATCC^{®} CCL-196^{™}, Manassas, VA, USA) was treated with each of the two selected sequences (SEQ ID NOs: 19 and 20) and the mouse SAMiRNA-amphiregulin of SEQ ID NO: 21 discovered through previous milestone studies, at treatment concentrations of 200 nM, 500 nM and 1,000 nM in cell culture media containing 10% FBS, and the expression inhibitory effects were additionally evaluated. As a result, it was confirmed again that SEQ ID NO: 20 exhibited the best expression inhibitory effect (FIG. 10b).

As shown in FIG. 10, two SAMiRNAs that most effectively inhibit amphiregulin gene expression were finally selected from 237 SAMiRNAs targeting mouse amphiregulin, and information on the sequences of the selected SAMiRNAs is shown in Table 9 below.

**[Table 8] Information on primer sequences for qPCR**

| Primer | Sequence |
|---|---|
| mGAPDH-F | AGGTCGGTGTGAACGGA TTTG (SEQ ID NO: 22) |
| mGAPDH-R | TGTAGACCATGTAGTTGAGGTCA (SEQ ID NO: 23) |
| mAREG-F | GAGGCTTCGACAAGAAAACG (SEQ ID NO: 24) |
| mAREG-R | ACCAATGTCATTTCCGGTGT (SEQ ID NO: 25) |

| | |
|---|---|
| (F denotes a forward primer, and R denotes a reverse primer) | |

**[Table 9] SAMiRNA sequences that effectively inhibit mouse amphiregulin expression**

| SEQ ID NO | Code Name | Position | Sense strand sequence |
|---|---|---|---|
| 19 | SAMi-mAREG#19 | 936-954 | AACGGGACTGTGCATGCCA |
| 20 | SAMi-mAREG#20 | 937-955 | ACGGGACTGTGCATGCCAT |
| 21 | SAMi-mAREG#21 | 1071-1089 | CAGTTGTCACTTTTTATGA |

### 9-1. Animal Testing Method

5-week-old BKS.Cg-*^{m}+*/*+ Lepr^{db}*/(KRIBB; Korea Institute of Bioscience and Biotechnology) were purchased and experiments were conducted using the mice. The purchased obese diabetic mice (db/db mice) were acclimated for 3 weeks and then fed rodent diet (2918C, Harlan, USA) for 8 weeks, and experiments were conducted using the mice under the following conditions. For comparison, 5-week-old non-obese normal mice (C57BL/6 mice) were purchased from Nara Biotech Co., Ltd. (Korea) and experiments were conducted using the mice. These mice were also acclimated for 3 weeks and fed rodent diet. All the mouse groups used in the experiments each consisted of 6 animals.

100 µl of PBS (C-9011, Bioneer, Korea) was administered to the normal control group, and the obese diabetic mice were divided into three groups to which 100 µl of PBS, 100 µl of SAMiRNA-Cont (5 mg/kg), and 100 µl of SAMiRNA-AREG (5 mg/kg) were respectively administered by subcutaneous injection three times a week. During the experiment period, the mice were housed in a constant environment at a temperature of 21 ± 2°C, a humidity of 55 ± 5%, 15 to 17/hour, an illuminance of 150 to 300 Lux, and a 12-hr light/12-hr dark cycle (turning on: 06:00, turning off: 18:00). After 8 weeks of drug administration, the mice were fasted for 16 hours and sacrificed.

### 9-2. Measurement of Body Weight, Food Intake and Water intake

Body weight, food intake and water intake were measured at predetermined time points twice a week.

### 9-2-1. Changes in Body Weight

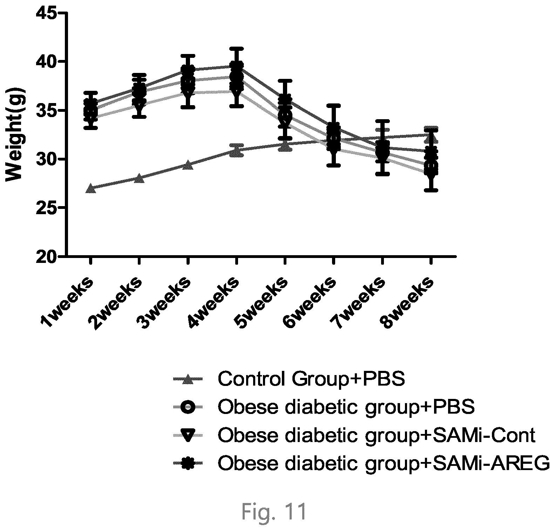

The body weight (g) of each mouse was measured at a predetermined time point twice a week. The body weight of the normal control group tended to gradually increase over time, and was 32.5±1.64 g at the time of mouse sacrifice. The three obese diabetic groups showed average body weights of 28.9±2.35 g (administration of PBS), 27.4±3.93 g (administration of SAMiRNA-Cont), and 29.7±4.12 g (administration of SAMiRNA-AREG), respectively (FIG. 11).

As a result of observing the body weight at week 8, there was no statistically significant difference in body weight between the groups, but all the obese diabetic mouse groups showed a slight weight loss compared to the normal control mice, and there was no statistically significant difference in body weight between the obese diabetic mouse groups, and the obese and diabetic group.

### 9-2-2. Changes in Food Intake and Water Intake

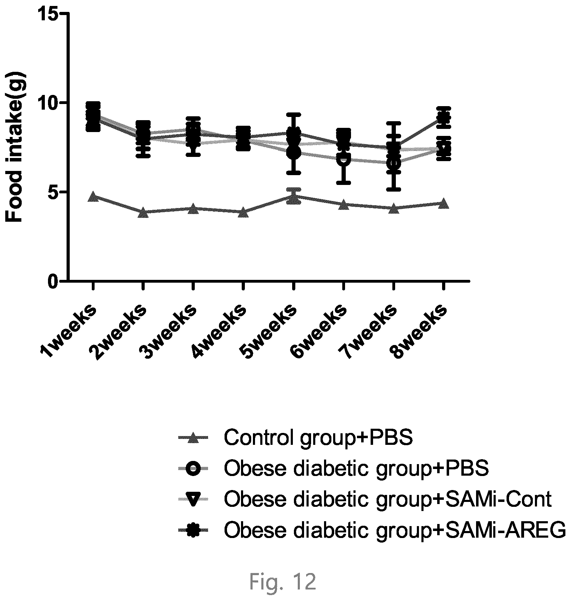

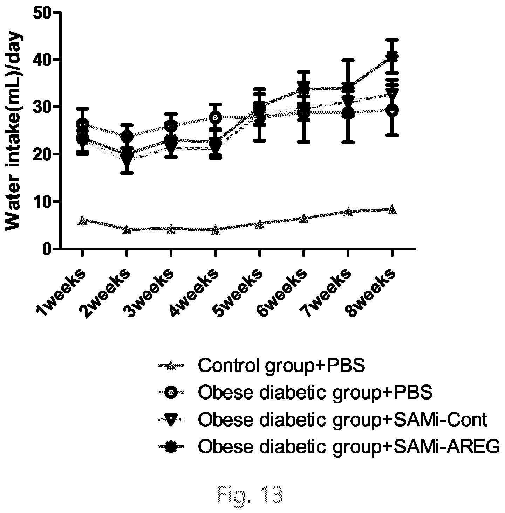

As a result of measuring mouse food intake, it was confirmed that the average daily food intake was about 4 g in the normal control group and about 8 g in all the obese diabetic groups, indicating that food intake more than doubled in the obese diabetic mice (FIG. 12). In addition, the water intake was about 5 mL/day in the normal control mice, but 20 to 35 mL/day in the obese diabetic mice, which was significantly higher (FIG. 13).

### 9-3. Changes in Fat Weight

For fat weight measurement, subcutaneous fat and visceral fat were extracted from the sacrificed mice, and their weights were measured, and the ratio of fat weight to mouse weight was evaluated.

The subcutaneous fat ratio was 1.56±0.36 in the normal control mice, 4.32±0.73 in the PBS-administered obese diabetic mice, 3.63±1.80 in the SAMiRNA-Cont-administered obese diabetic mice, and 3.15±0.58 in the SAMiRNA-AREG-administered obese diabetic mice. Thereby, it could be confirmed that, in the obese diabetic mice, the weight of subcutaneous fat was significantly reduced by reducing the expression level of amphiregulin according to the present invention (FIG. 14).

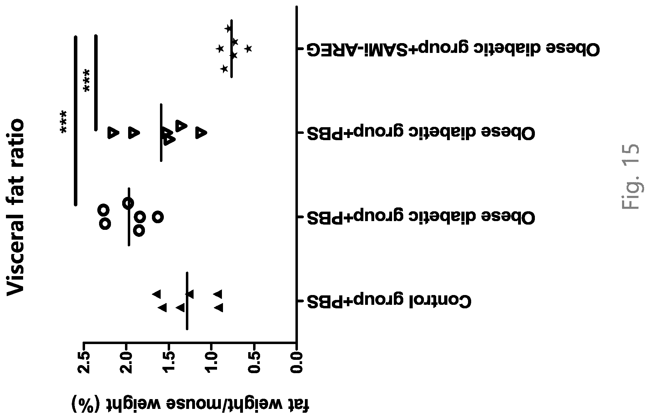

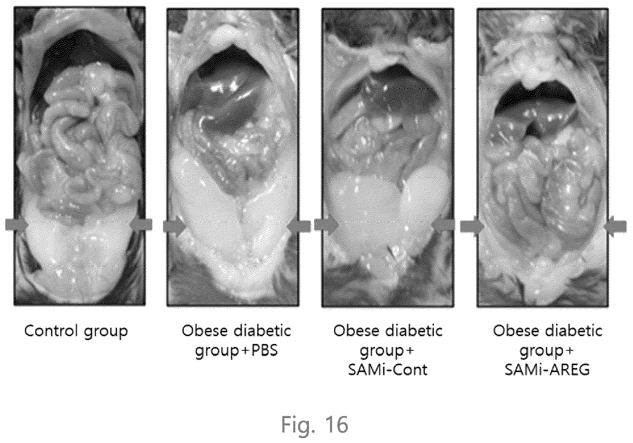

The visceral fat ratio was 1.28±0.31 in the normal control mice, 1.97±0.25 in the PBS-administered obese diabetic mice, 1.59±0.38 in the SAMiRNA-Cont-administered obese diabetic mice, and 0.76±0.12 in the SAMiRNA-AREG-administered obese diabetic mice. Thereby, it could be confirmed that, in the obese diabetic mice, the weight of visceral fat was significantly reduced by reducing the expression level of amphiregulin according to the present invention (FIGS. 15 and 16).

### 9-4. Fasting Blood Glucose Level Measurement and Serum Glucose Level measurement

Fasting blood glucose levels (mg/dL) were measured using a simple blood glucose meter (Accu-ChekTM Active, Roche, Germany) one week before mouse sacrifice, and serum glucose levels were measured by KP&T Co., Ltd.

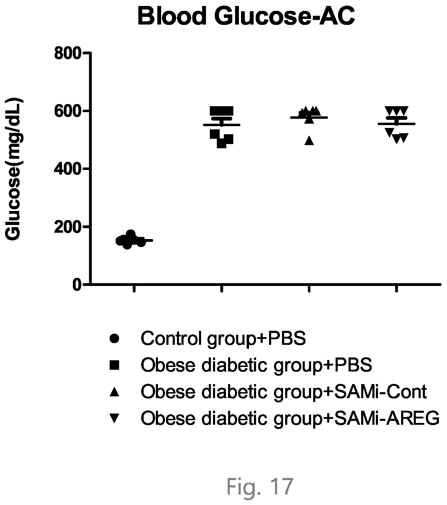

The blood glucose levels were 153.2±12.07 in the normal control mice, 551.8±53.73 on average in the PBS-administered obese diabetic mice, 577.3±40.25 on average in the SAMiRNA-Cont-administered obese diabetic group, and 555.7±49.15 on average in the SAMiRNA-AREG-administered obese diabetic group (FIG. 17).

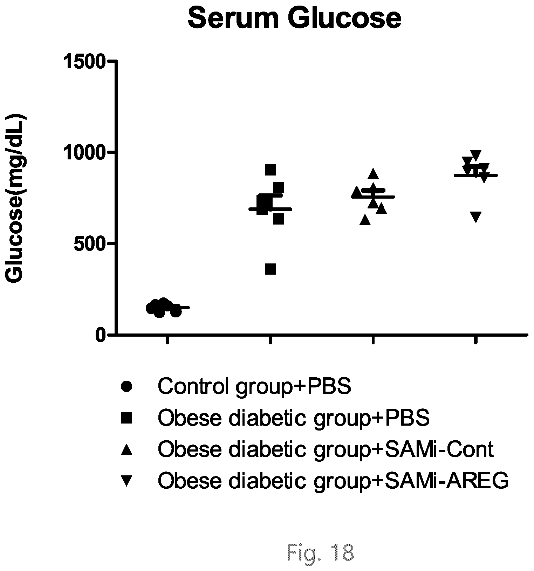

The serum glucose levels (mg/dL) were 149.6120.4 in the normal control mice, 689.1±185.4 on average in the PBS-administered obese diabetic mice, 755.5±89.4 in the SAMiRNA-Cont-administered obese diabetic group, and 874.3±119.4 on average in the SAMiRNA-AREG-administered obese diabetic group (FIG. 18).

Therefore, it was confirmed that the decrease in the expression level of amphiregulin in obese diabetic mice according to the present invention had no significant effect on the control of fasting blood glucose levels or serum glucose levels in the diabetic animal models.

### Example 10. Evaluation of Anti-Obesity Effect Using High-Fat Diet Obesity Model

In order to evaluate the anti-obesity effect of an amphiregulin expression inhibitor, the amphiregulin expression inhibitor SAMiRNA-AREG (SAMi-mAREG#20) was administered to a high-fat-diet-induced obesity model, and the effect thereof on fat reduction was observed. After administration of high-fat diet, the drug was administered three times a week for 5 weeks, and then various indicators were analyzed.

### 10-1. Analysis of Amphiregulin Expression Level After Administration of High-Fat Diet

5-week-old C57BL/6 mice (Nara Biotech Co., Ltd.) were purchased and experiments were conducted using the mice. The purchased mice were acclimated for 1 week and then fed a 60 kcal% fat diet (D12492, Research Diets, Inc.) for 6 weeks. Next, epididymal fat was extracted and mRNA levels therein were measured.

Total RNA extraction from adipose tissue was performed using a combination of QIAzol lysis reagent (QIAZEN., GER) and RNeasy Mini Kit (QIAZEN., GER). 100 mg of adipose tissue was added to 1 ml of QIAzol lysis reagent, homogenized and then incubated for 5 minutes at room temperature. After centrifugation at 12,000g at 4°C for 10 minutes, the lower sample excluding the upper fat monolayer was collected. 200 µl of chloroform (Sigma-Aldrich, USA) was added to and mixed well with the sample, and the mixture was kept at room temperature for 3 minutes, followed by centrifugation at 12,000g at 4°C for 30 minutes. Among the three separated phases, the uppermost layer was collected and 70% EtOH was added thereto and mixed well therewith at a ratio of lysate: 70% EtOH = 1: 1, and then loaded into the RNeasy spin column of the RNeasy Mini Kit. The subsequent process was performed according to the manufacturer's protocol. The extracted RNA was quantified using a spectrophotometer and the purity thereof was evaluated.

**[Table 9] Information on primer sequences for qPCR**

| Name | F primer | R primer | Probe |
|---|---|---|---|
| Areg | AGTAGTAGCTGTCACTATCTTT GTC (SEQ ID NO: 51) | CCCGTTTTCTTGTCGAAGC (SEQ ID NO: 52) | CCTCGCAGCTATTGGCATCGGCA (SEQ ID NO: 53) |
| Tfrc | AAACTTGCCCAAGTATTCTCAG (SEQ ID NO: 54) | ATGAAAGGTATCCCTCCAA CC (SEQ ID NO: 55) | TGCCAGCTGGACTGCAGGCGACT (SEQ ID NO: 56) |

| | | | |
|---|---|---|---|
| (F denotes a forward primer, and R denotes a reverse primer) | | | |

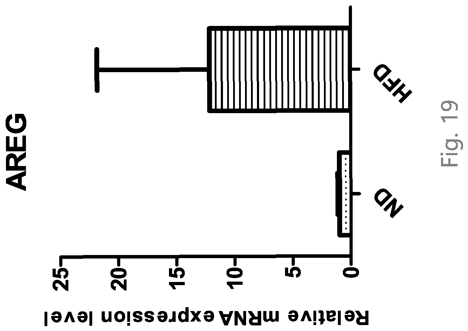

As a result, it was confirmed that the expression level of amphiregulin increased 12-fold compared to the normal control group by administration of the high-fat diet (FIG. 19).

### 10-2. Animal Testing Method

5 week-old C57BL/6 mice (Nara Biotech Co., Ltd.) were purchased and experiments were performed using the mice. The purchased mice were acclimated for 1 week and then fed a 60 kcal% fat diet (D12492, Research Diets, Inc.) for 1 week, and then experiments were conducted using the mice under the following conditions. For comparison, non-obese normal mice were fed rodent diet (2918C, Harlan, USA) under the same conditions, and experiments were conducted using the mice under the following conditions. For experiments, the mice were divided into a normal control group (ND+PBS) and a test substance-administered group (HFD+SAMi-AREG), each consisting of 6 animals, and a negative control group (HFD+PBS) consisting of 5 animals.

100 µl of PBS (LB 001-02, WELGENE, Korea) was administered to the normal control group (ND+PBS) and the negative control group (HFD+PBS), and 100 µl of SAMiRNA-AREG (5 mg/kg) was administered to the test substance-administered group (HFD+SAMi-AREG) by subcutaneous injection three times a week.

During the experiment period, the mice were housed in a constant environment at a temperature of 21 ± 2°C, a humidity of 55 ± 5%, 15 to 17/hour, an illuminance of 150 to 300 Lux, and a 12-hr light/12-hr dark cycle (turning on: 06:00, turning off: 18:00). After 8 weeks of drug administration, the mice were fasted for 16 hours and sacrificed.

### 10-3. Measurement of Body Weight, Food Intake and Water intake

Body weight, food intake and water intake were measured at predetermined time points twice a week.

### 10-3-1. Changes in Body Weight

The body weight (g) of each mouse was measured at a predetermined time point twice a week. The body weight of the negative control group tended to increase due to the high-fat diet over time after PBS administration, and was 37.3±1.20 g at the time of mouse sacrifice. The body weight (g) was 26.57±0.36 on average in the PBS-administered normal control group, and 33.21±1.16 on average in the SAMiRNA-AREG-administered group. As a result of observing the body weight at weeks 4 and 5, there was a statistically significant difference in the body weight between the test substance-administered group and the negative control group.

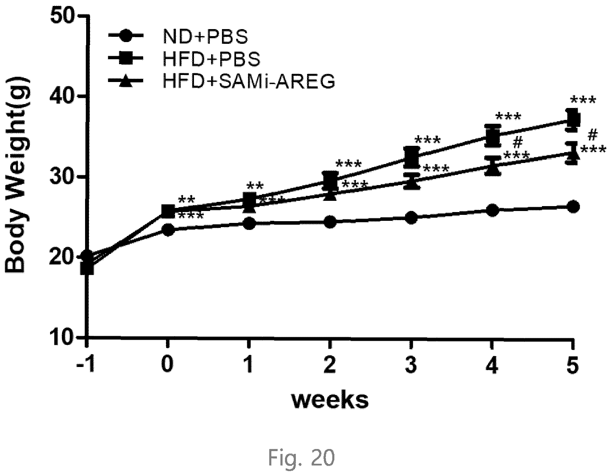

Compared to the body weight of the normal control mice, the body weight of the mice in the high-fat diet-induced negative control group increased significantly, whereas the body weight of the mice in the test substance-administered group decreased, which was statistically significant (FIG. 20).

### 10-3-2. Changes in Food Intake and Water Intake

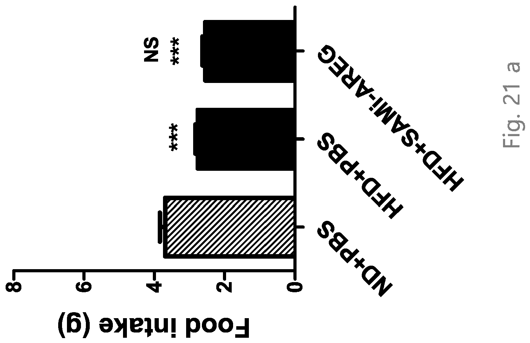

As a result of measuring mouse food intake, it was confirmed that the average daily food intake was 3.69±0.15 g in the normal control group, 2.76±0.06 g in the negative control group, and 2.55±0.08 g in the test substance-administered group, indicating that the food intake of the high-fat-diet group was smaller than that of the negative control group, and there was no significant difference in food intake between the negative control group and the test substance-administered group (FIG. 21a).

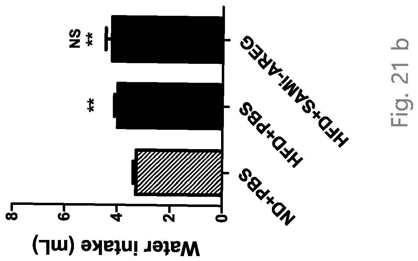

As a result, as a result of measuring mouse water intake (mL), it was confirmed that the daily average water intake was 3.27±0.10 in the normal control group, 4.00±0.12 in the negative control group, and 4.22±0.24 in the test substance-administered group, indicating that the water intake of the high-fat-diet group was greater than that of the negative control group, and there was no significant difference in water intake between the negative control group and the test substance-administered group (FIG. 21b).

### 10-4. Food Efficiency Ratio (%)

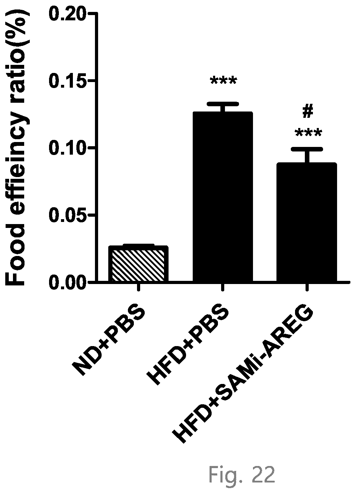

As a result of measuring food efficiency ratio by dividing the weight gain (g/day) of each mouse by the food intake (g/day), it was confirmed that the food efficiency ratio was 0.02±0.001 in the normal control group, 0.12±0.007 in the negative control group, and 0.08±0.011 in the test substance-administered group, indicating that, even though the food efficiency ratio in both the negative control group and the test substance-administered group increased compared to that in the normal control group, the food efficiency ratio in the test substance-administered group was significantly lower than that in the negative control group (FIG. 22).

### 10-5. Changes in Fat Weight

To measure the weight of fat, the subcutaneous fat pad, epididymal fat pad, perirenal fat pad, and mesenteric fat pad were extracted from sacrificed mice, and the weights thereof were measured, and the ratio of fat weight to mouse weight was evaluated.

The mouse subcutaneous fat pad weight (g) was 0.38±0.02 in the normal control group, 1.74±0.12 in the negative control group, and 1.05±0.14 in the test substance-administered group, and the epididymal fat pad weight (g) was 0.52±0.03 in the normal control group, 2.32±0.09 in the negative control group, and 1.79±0.16 in the test substance-administered group. In addition, the perirenal fat pad weight (g) was 0.20±0.02 in the normal control group, 0.86±0.04 in the negative control group, and 0.67±0.08 in the test substance-administered group, and the mesenteric fat pad weight (g) was 0.25±0.01 in the normal control group, 0.66±0.09 in the negative control group, and 0.45±0.04 in the test substance-administered group (FIG. 23a) .

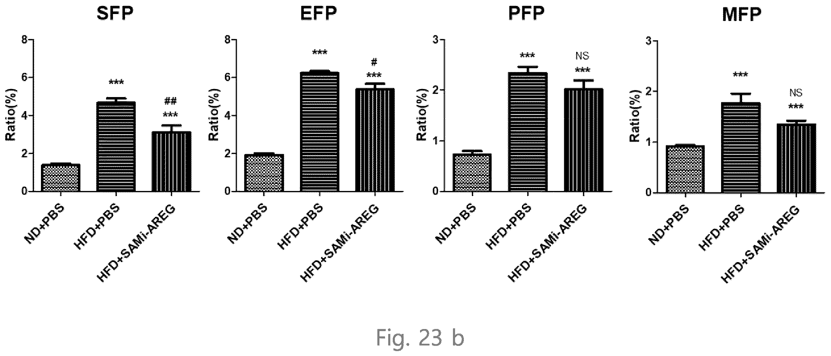

The ratio (%) of the mouse subcutaneous fat pad weight was 1.39±0.08 in the normal control group, 4.65±0.23 in the negative control group, and 3.11±0.35 in the test substance-administered group, and the ratio (%) of the epididymal fat pad weight was 1.91±0.10 in the normal control group, 6.23±0.09 in the negative control group, and 5.36±0.29 in the test substance-administered group. In addition, the ratio (%) of the perirenal fat pad weight was 0.73±0.06 in the normal control group, 2.33±0.13 in the negative control group, and 2.01±0.17 in the test substance-administered group, and the ratio (%) of the mesenteric fat pad weight was 0.91±0.02 in the normal control group, 1.76±0.19 in the negative control group, and 1.35±0.07 in the test substance-administered group (FIG. 23b) .

The mouse fat was divided into subcutaneous fat and visceral fat (epididymal fat, perirenal fat, and mesenteric fat), and the weight (g) and the ratio (%) of the weight were measured.

For the mouse subcutaneous fat, the weight was 0.38±0.02 in the normal control group, 1.74±0.12 in the negative control group, and 1.05±0.14 in the test substance-administered group, and the ratio of the weight was 1.39±0.08 in the normal control group, 4.65±0.23 in the negative control group, and 3.11±0.35 in the test substance-administered group.

For the visceral fat, the weight was 0.97±0.06 in the normal control group, 3.85±0.18 in the negative control group, and 2.92±0.27 in the test substance-administered group, and the ratio of the weight was 3.56±0.17 in the normal control group, 10.34±0.16 in the normal control group, and 8.73±0.50 in the test substance-administered group.

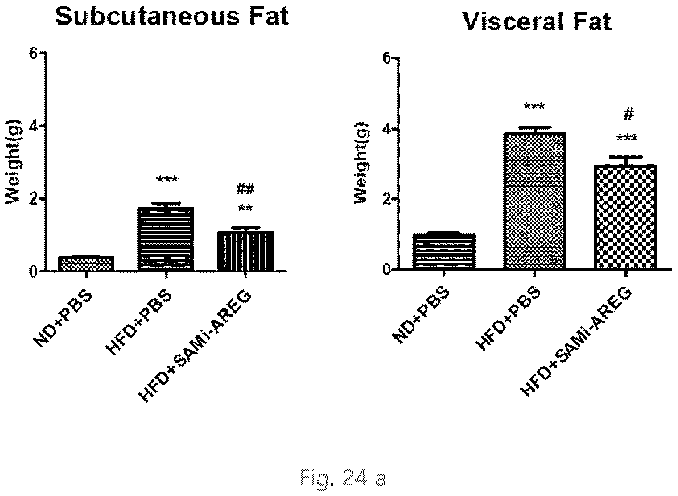

As a result, it could be confirmed that the weight and weight ratio of subcutaneous fat and the weight and weight ratio of visceral fat significantly increased in the negative control group compared to the normal control group, and this increase in the weight and weight ratio in the negative control group significantly decreased in the test substance-administered group (FIGS. 24a and 24b).

### 10-6. Micro CT Analysis

For Micro-CT analysis of mouse fat (Korea Basic Science Institute, Gwangju Center), two animals with a median body weight were selected from each group. The selected mice were photographed, and the volumes of subcutaneous fat and visceral fat were graphed.

The volume (mm³) of subcutaneous fat was 16611289.5 in the normal control group, 6356±217 in the negative control group, and 4040±284.5 in the test substance-administered group, and the volume (mm³) of visceral fat was 1069±90 in the normal control group, 3782±7 in the negative control group, and 2623±166 in the test substance-administered group.

As a result, it could be confirmed that the volumes of subcutaneous fat and visceral fat increased in the negative control group compared to the normal control group, and decreased in the test substance-administered group compared to the negative control group (FIG. 25).

### 10-7. Histopathological Examination of Adipose Tissue

Subcutaneous fat pad, epididymal fat pad, perirenal fat pad, and mesenteric fat pad were extracted from the mice, fixed in 10% neutral buffered formalin (Sigma, HT50-1-640) for 24 hours or more, dehydrated with ethanol, and then cleared with xylene three times. Then, the tissue samples were infiltrated and embedded in liquid paraffin, thus preparing paraffin blocks. Next, the 5 um-thick tissue sections were deparaffinized and rehydrated, and the nuclei were stained with Harris hematoxylin staining solution for 5 min, followed by counter staining with eosin solution. After staining, a mounting solution was dropped onto the tissue slides which were then covered with cover glass and hardened. The tissue slides hardened with the preservation solution were photographed at 200x magnification using an inverted microscope (Nikon Eclipse TS2), and the area of adipocytes was calculated.

For the subcutaneous fat pad, the adipocyte area (mm²) was 913.8±129.9 in the normal control group, 3,575.0±346.7 in the negative control group, and 1,337.0±211.1 in the test substance-administered group, and for the epididymal fat pad, the adipocyte (mm²) was 950.6±73.2 in the normal control group, 1,598.0±99.6 in the negative control group, and 1347.0±100.1 in the test substance-administered group. For the perirenal fat pad, the adipocyte area (mm²) was 1,734.0±158.8 in the normal control group, 5,365.0±190.4 in the negative control group, and 2,516.0±288.3 in the test substance-administered group, and for the mesenteric fat pad, the adipocyte area (mm²) was 1,552.01680.3 in the normal control group, 3,495.0±425.3 in the negative control group, and 1,436.0±163.3 in the test substance-administered group.

As a result, it could be confirmed that the adipocyte area increased in the negative control group compared to the normal control group, and decreased in the test substance-administered group compared to the negative control group (FIG. 26).

### 10-8. Histopathological Examination of Liver Tissue

Livers were extracted from the mice, fixed in 10% neutral buffered formalin (Sigma, HT50-1-640) for more than 24 hours, and subjected to H&E staining and Oil red O analysis, followed by examination.

### 10-8-1. H&E Staining

H&E (Hematoxylin & Eosin) staining was performed to observe the overall morphology of the liver tissue obtained after fixation in neutral buffered formalin and the degree of lipid accumulation in the liver tissue. Tissue samples were prepared by paraffin infiltration. After dehydration with ethanol, the tissue samples were cleared with xylene three times. The tissue blocks were cut into 5 um-thick sections using a microtome, and the sections were dried in a slide dryer for 1 hour, deparaffinized with xylene, treated with ethanol, and then hydrated. Nuclei were first stained with Harris hematoxylin staining solution for 5 minutes, followed by counter staining with eosin. After staining, a mounting solution was dropped onto the tissue slides which were then covered with cover glass and hardened. The tissue slides hardened with the preservative solution were observed using a microscope.

### 10-8-2. Oil Red O Staining

The liver tissues obtained after fixing in neutral buffered formalin were infiltrated in 30% sucrose solution. After washing with PBS solution, the tissues were dehydrated and fixed in optimal cutting temperature (O.C.T) compound (SAKURA, U.S.A.), a frozen tissue embedding agent, to obtain frozen tissue blocks. The fixed tissues were sectioned to a thickness of 14 um using a Cryostat CM3050 S (Leica) to obtain tissue samples. The lipid accumulation inhibitory effect of AREG was evaluated using Oil Red O staining, a special staining method that can determine the degree of lipid accumulation by reacting specifically and sensitively with a lipid component and displaying a red color. After fixing in 10% neutral buffered formalin (Sigma, HT50-1-640), the fixative was removed, and the samples were washed with 100% propylene glycol and stained with Oil red O solution that reacts specifically with intracellular lipid droplets. After staining, a mounting solution was dropped onto the tissue slides which were then covered with a coverslip and hardened. The tissue slides hardened with the preservative solution were observed using a microscope.

### 10-8-3. Changes in Liver Fat

As a result of performing quantitative analysis after Oil Red O analysis, it was confirmed that adipogenesis increased by 6.6 times in the negative control group compared to the normal control group, and increased 2.8 times in the test substance-administered group compared to the normal control group. Thus, it could be seen that adipogenesis was inhibited in the test substance-administered group (FIG. 27).

### 10-9. Gene Expression Analysis

The expression levels of amphiregulin in the subcutaneous fat pad, epididymal fat pad and peripheral fat pad from the mice were measured. Total RNA extraction from adipose tissue was performed using a combination of TRI Reagent (MRC Inc., USA) and Universal RNA extraction kit (BIONEER, Korea). 100 mg of adipose tissue was added to 1 ml of TRI Reagent, homogenized and then incubated for 5 minutes at room temperature. After centrifugation at 12,000g and 4°C for 10 minutes, the lower sample excluding the upper fat monolayer was collected. 200 µl of chloroform (Sigma-Aldrich, USA) was added to and mixed well with the sample, and the mixture was kept at room temperature for 3 minutes, followed by centrifugation at 12,000g at 4°C for 30 minutes. Among the three separated phases, the uppermost layer was collected and isopropanol was added thereto and mixed well therewith at a ratio of lysate:isopropanol = 400: 300, and then loaded into the AccuPrep^{®} Binding Column-III of the Universal RNA extraction kit. The subsequent processes were performed according to the manufacturer's protocol. The extracted RNA was quantified using a spectrophotometer, the purity thereof was evaluated, and the degree of degradation thereof was determined through RNA gel-electrophoresis.

**[Table 10] Information on primer sequences for qPCR**

| Name | F primer | R primer |
|---|---|---|
| Areg | GAGGCTTCGACAAGAAAACG (SEQ ID NO: 57) | ACCAATGTCATTTCCGGTGT (SEQ ID NO: 58) |
| Gapdh | AGGGTGGAGCCAAAAGGGTC (SEQ ID NO: 59) | GGCTAAGCAGTTGGTGGTGC (SEQ ID NO: 60) |

| | | |
|---|---|---|
| (F denotes a forward primer, and R denotes a reverse primer) | | |

### 10-9-1. Amphiregulin mRNA Levels in Adipose Tissue

The amphiregulin mRNA level in the mouse subcutaneous fat pad increased by 1.8 times in the negative control group compared to the normal control group, but decreased by 1.6 times in the test substance-administered group compared to the negative control group.

The amphiregulin mRNA level in the epididymal fat pad increased by 3.2 times in the negative control group compared to the normal control group, but decreased by 1.3 times in the test substance-administered group compared to the negative control group.

The amphiregulin mRNA level in the perirenal fat pad increased by 3.7 times in the negative control group compared to the normal control group, but decreased by 1.5 times in the test substance-administered group compared to the negative control group (FIG. 28).

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The pharmaceutical composition according to the present invention has an excellent effect of reducing visceral fat, as well as subcutaneous fat, and thus may be effectively used for the treatment and prevention of cardiovascular disease, metabolic disease, diabetes, and various other diseases in which complications caused by visceral fat become a problem.

## Claims

1. A pharmaceutical composition for treating or preventing obesity comprising any one selected from the group consisting of:
(i) an amphiregulin-specific double-stranded oligonucleotide, which comprises any one sequence selected from the group consisting of SEQ ID NOs: 10, 11 and 12, and an anti-sense strand comprising a sequence complementary to that of the sense strand;
(ii) an amphiregulin-specific double-stranded oligonucleotide structure comprising a structure represented by the following Structural Formula (1):
[Structural Formula (1)] A-X-R-Y-B
wherein A represents a hydrophilic compound, B represents a hydrophobic compound, X and Y each independently represent a simple covalent bond or a linker-mediated covalent bond, and R represents the amphiregulin-specific double-stranded oligonucleotide (i); and
(iii) nanoparticles comprising the amphiregulin-specific double-stranded oligonucleotide structure (ii).

2. The pharmaceutical composition according to claim 1, wherein the sense strand or the antisense strand consists of 19 to 31 nucleotides.

3. The pharmaceutical composition according to claim 1, wherein the oligonucleotide is siRNA, shRNA or miRNA.

4. The pharmaceutical composition according to claim 1, wherein the sense strand or the antisense strand is independently DNA or RNA.

5. The pharmaceutical composition according to claim 1, wherein the sense strand or the antisense strand of the double-stranded oligonucleotide comprises a chemical modification.

6. The pharmaceutical composition according to claim 5, wherein the chemical modification is any one or more selected from the group consisting of:
modification in which an OH group at the 2' carbon position of a sugar structure in one or more nucleotides is substituted with any one selected from the group consisting of a methyl group (-CH₃), a methoxy group (-OCH₃), an amine group (-NH₂), fluorine (-F), a -O-2-methoxyethyl group, an -O-propyl group, an -O-2-methylthioethyl group, an -O-3-aminopropyl group, an -O-3-dimethylaminopropyl group, an -O-N-methylacetamido group, and an -O-dimethylamidooxyethyl group;
modification in which oxygen in a sugar structure in nucleotides is substituted with sulfur; modification of a bond between nucleotides into any one bond selected from the group consisting of a phosphorothioate bond, a boranosphophate bond and a methyl phosphonate bond;
modification to PNA (peptide nucleic acid), LNA (locked nucleic acid) or UNA (unlocked nucleic acid); and
modification to a DNA-RNA hybrid.

7. The pharmaceutical composition according to claim 1, wherein at least one phosphate group is bound to the 5' end of the antisense strand of the double-stranded oligonucleotide.

8. The pharmaceutical composition according to claim 1, wherein the amphiregulin-specific double-stranded oligonucleotide structure comprises a structure represented by the following Structural Formula (2):
[Structural Formula (2)] A-X-S-Y-B AS
wherein S and AS respectively represent the sense strand and the antisense strand of the double-stranded oligonucleotide according to claim 1, and A, B, X and Y are as defined in claim 1.

9. The pharmaceutical composition according to claim 8, wherein the amphiregulin-specific double-stranded oligonucleotide structure comprises a structure represented by the following Structural Formula (3) or (4) :
[Structural Formula (3)] A - X - 5' S 3' - Y - B AS
[Structural Formula (4)] A - X - 3' S S' - Y - B AS
wherein A, B, X, Y, S and AS are the same as defined in claim 8, and 5' and 3' respectively represent the 5' end and the 3' end of the sense strand of the double-stranded oligonucleotide sense strand.

10. The pharmaceutical composition according to claim 1, wherein the hydrophilic compound is selected from the group consisting of polyethylene glycol (PEG), polyvinylpyrrolidone and polyoxazoline.

11. The pharmaceutical composition according to claim 1, wherein the hydrophilic compound has a structure represented by the following Structural Formula (5) or (6) :
[Structural Formula (5)] (A'ₘ-J)ₙ
[Structural Formula (6)] (J-A'ₘ)ₙ
wherein A' represents a hydrophilic monomer, J represents a linker that connects m hydrophilic monomers together or connects m hydrophilic monomers with the double-stranded oligonucleotide, m is an integer ranging from 1 to 15, n is an integer ranging from 1 to 10,
the hydrophilic monomer (A') is any one compound selected from among the following compound (1) to compound (3), and the linker (J) is selected from the group consisting of -PO₃⁻-, -SO₃- and - CO₂-: 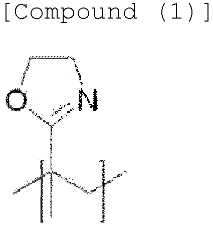
wherein G is selected from the group consisting of O, S and NH;

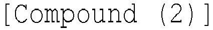

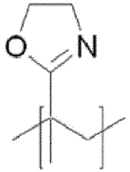

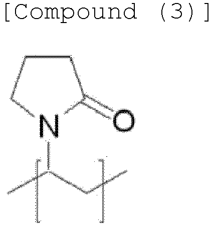

12. The pharmaceutical composition according to claim 11, wherein the amphiregulin-specific double-stranded oligonucleotide structure has a structure represented by the following Structural Formula (7) or Structural Formula (8) :
[Structural Formula (7)] (A'ₘ-J)ₙ -X-R-Y-B
[Structural Formula (8)] (J-A'ₘ)ₙ-X-R-Y-B

13. The pharmaceutical composition according to claim 1, wherein the hydrophilic compound has a molecular weight of 200 to 10,000.

14. The pharmaceutical composition according to claim 1, wherein the hydrophobic compound has a molecular weight of 250 to 1,000.

15. The pharmaceutical composition according to claim 14, wherein the hydrophobic compound is any one selected from the group consisting of a steroid derivative, a glyceride derivative, glycerol ether, polypropylene glycol, a C₁₂-C₅₀ unsaturated or saturated hydrocarbon, diacylphosphatidylcholine, a fatty acid, a phospholipid, lipopolyamine, a lipid, tocopherol, and tocotrienol.

16. The pharmaceutical composition according to claim 15, wherein the steroid derivative is any one selected from the group consisting of cholesterol, cholestanol, cholic acid, cholesteryl formate, cholestanyl formate, and cholestanyl amine.

17. The pharmaceutical composition according to claim 15, wherein the glyceride derivative is any one selected from the group consisting of mono-glyceride, di-glyceride, and triglyceride.

18. The pharmaceutical composition according to claim 1, wherein the covalent bond represented by each of X and Y is either a nondegradable bond or a degradable bond.

19. The pharmaceutical composition according to claim 18, wherein the nondegradable bond is an amide bond or a phosphate bond.

20. The pharmaceutical composition according to claim 18, wherein the degradable bond is any one selected from the group consisting of a disulfide bond, an aciddegradable bond, an ester bond, an anhydride bond, a biodegradable bond, and an enzyme-degradable bond.

21. The pharmaceutical composition according to claim 1, wherein the nanoparticle is composed of a mixture of double-stranded oligonucleotide structures comprising double-stranded oligonucleotides comprising different sequence.

22. The pharmaceutical composition according to claim 1, wherein the obesity is visceral fat-type obesity caused by diabetes.

23. The pharmaceutical composition according to claim 1, wherein the pharmaceutical composition exhibits one or more of the following effects:
(i) loss of body weight,
(ii) reduction of food efficiency ratio,
(iii) reduction of subcutaneous fat,
(iv) reduction of visceral fat,
(v) reduction of adipocyte area, and
(vi) inhibition of liver adipogenesis.

24. The pharmaceutical composition according to claim 1, wherein the pharmaceutical composition exhibits an effect of preventing or treating obesity by inhibiting amphiregulin expression in adipose tissue.

25. A lyophilized formulation comprising the pharmaceutical composition according to claim 1.
